# EUROPEAN PATENT SPECIFICATION

(11) **EP 0 839 055 B1**
(45) Date of publication and mention of the grant of the patent: **18.05.2005**
(21) Application number: 96921484.0
(22) Date of filing: 07.06.1996
(51) Int. Cl.: A61K 49/00, C12N 5/10, A61K 47/48, A61K 38/51, C12N 9/88

(54) **MODIFIED METHIONINASE, ITS USE, RECOMBINANT HOST CELLS PRODUCING HIGH LEVELS OF METHIONINASE AND METHOD FOR PURIFYING METHIONINASE**
MODIFIZIERTE METHIONINASE, DEREN VERWENDUNG, REKOMBINANTE WIRTsZELLEN DIE GROSSE MENGEN VON METHIONINASE PRODUZIEREN UND VERFAHREN ZUR AUFREINIGUNG VON METHIONINASE
METHIONINASE MODIFIEE, SON UTILISATION, CELLULES HOTE RECOMBINANTES PRODUISANT DE GRANDES QUANTITES DE METHIONINASE ET METHODE DE PURIFICATION DE LA METHIONINASE

(30) Priority: 07.06.1995 US 486519; 03.05.1996 US 642541
(43) Date of publication of application: 06.05.1998
(73) Proprietor: ANTICANCER, INC., San Diego, CA 92111 (US)
(72) Inventor: TAN, Yuying, San Diego, CA 92111 (US); LISHKO, Valeryi, Shaker Heights, OH 44122 (US)
(74) Representative: W.P. Thompson & Co.
(86) International application number: PCT/US1996/009935
(87) International publication number: WO 1996/040284

(56) References cited:
- WO-A-94/11535
- US-A- 4 298 590
- US-A- 5 028 420
- US-A- 5 487 984
- ESAKI N ET AL: "L- methionine gamma- lyase from Pseudomonas putida and Aeromonas." METHODS IN ENZYMOLOGY, (1987) 143 459-65. , XP001031134
- JOURNAL OF THE NATIONAL CANCER INSTITUTE, Volume 82, No. 20, 17 October 1990, F. BREILLOUT et al., "Methionine Dependency of Malignant Tumors: A Possible Approach for Therapy", pages 1628-1632, XP001038180.
- Inada Y. et al: 'Biomedical and biotechnological applications of PEG- and M-modified proteins', Trends in Biotechnology, 13(3):86-91 (1995-03-00)

## Description

### Technical Field

The present invention relates to modified methioninase compositions, methods of purification of methioninase, methods of coupling methioninase to a polymer such as PEG, use of methioninase in antimethionine and anti-homocysteine chemotherapy. More particularly, the invention includes the use of modified methioninase in cancer therapy, cardiovascular therapy, and tumor imaging and diagnosis. The present invention further relates to recombinant DNA technology. The present invention particularly provides high expression modules that encode methioninase.

### Background

Therapeutic drug-based treatment of cancer is directed at the use of medicinals which selectively inhibit or kill the cancer cells while not harming normal tissue function beyond acceptable amounts. The difficulty with conventional chemotherapy has been the toxicity of therapeutic drugs for normal tissue.

Many tumors have been shown to have absolute requirement for methionine in a variety of cell types and evaluated tumor tissues, including tumors of the colon, breast prostate, ovary, kidney, larynx melanoma, sarcoma, lung, brain, stomach and bladder as well as leukemias and lymphomas. Methionine dependence has been defined as an inability of tumors to grow when methionine is replaced by homocysteine in the growth medium. See, for example, Chello *et al*., Cancer Res., 33:1898-1904, 1973; and Hoffman, Anticancer Res., 5:1-30, 1985.

Methionine depletion has been shown to selectively synchronize methionine-dependent tumor cells into late S/G, phase of the cell cycle. Hoffman et al, Proc. Natl. Acad. Sci. USA, 77:7306-7310, 1980. Using the combination of methionine deprivation, followed by repletion of methionine coupled with exposure to an antimitotic agent, termed antimethionine chemotherapy, tumor cells have been selectively eliminated from cocultures of normal and tumor cells, resulting in cultures of normal cells proliferating vigorously. Stern *et al*., J. Natl. Cancer Inst., 76:629-639, 1986.

However, in order for methionine-dependent chemotherapy to be conducted *in vivo,* it is necessary to have a means to effectively deplete serum of circulating methionine. Methionine depletion methods have not been described that reduce circulating methionine levels *in vivo* in a manner sufficient to be effective in antitumor therapies.

Methioninase, an enzyme which degrades methionine, has been purified from a variety of bacterial sources, and has been reported to slow the rate of tumor cell proliferation *in vitro*. Kreis *et al*., Cancer Res., 33:1862-1865, and 1866-1869, 1973; Tanaka *et al*., FEBS Letters, 66:307-311 1976; Ito *et al*., J. Biochem. 79:1263-1272, 1976; and Nakayama *et al*., Agric. Biol. Chem. 48:2367-2369, 1984.

Kreis *et al*., Cancer Res. 33:1866-1869, 1973, have described the use of highly impure methioninase preparations isolated from *Clostridium sporgenes* at 1150 units/kg/day to inhibit growth of carcinosarcoma cells implanted in a mouse model. Although the enzyme apparently reduced primary tumor cell growth, it was not reported to reduce the T/C (treated versus control) ratio of tumor diameter below 50%, and was not reported to have any effect on metastasis. The authors also indicated that tumor specificity of the methioninase cannot be expected without other unspecified interventions, and further do not comment on the possibly that endotoxin, or other components of the impure preparation, were responsible for the effects observed. The only toxicity studies reported were absence of animal body weight loss after the duration of the treatment, and negative gross examination for toxicity. Further, the authors report that the enzyme had a serum half life of 4 hours. The enzyme preparation was only semi-pure and contained significant amounts of endotoxin complicating the interpretation of the results.

Further, Kreis was unable to reduce serum methionine levels to below about 8% of control. his is likely attributable to the high Kₘ (approx. 90mM) of the *Clostridium* enzyme. The methioninase preparation was reported to be unstable and only a 2% recovery (yield) could be achieved in its purification.

Kreis *et al*., Cancer Res. 33:1866-1869, 1973, further reported the use of a methionine-free diet as a means to deplete methionine as an antitumor therapy. However, the authors reported that the diet did not slow tumor growth as effectively as the use of an impure preparation of methioninase and resulted in the undesirable side effect of continuous loss of weight of the animal. The authors did not report the use of methionine deficient diets combined with methioninase treatment, and did not study cell synchronization.

The priority applications of the present invention disclose effective chemotherapy of tumors directed at effectively reducing the amount of methionine as to provide a beneficial antitumor effect without deleterious injury using methioninase. The present invention improves the disclosed therapeutic and diagnostic methods and composition by providing a source for producing commercially viable quantities of highly pure recombinant methioninase.

### Brief Description of the Invention

It has now been discovered that methioninase, either in PEGylated form or as produced and purified as a highly pure, endotoxin free recombinant form, can deplete levels of methionine in mammals without harm, can be effectively used to selectively inhibit tumor growth and further can be used to selectively arrest and thereby synchronize tumor cells for antimitotic chemotherapy.

It has also been discovered that methionine depletion is most effective when several different methods are used synergistically to substantially reduce effective methionine levels. These methods include methionine starvation using a methionine-free diet, competitive inhibition of methionine by use of inhibitors that compete with methionine for methionine-utilizing enzymes, chemotherapeutic agents that take advantage of the specific tumor selective cell-cycle block induced by methionine depletion and combinations thereof.

Thus, described herein is a method for inhibiting tumor cell growth comprising contacting a population of tumor cells with a therapeutically effective amount of methioninase as herein defined for a time period sufficient to induce cell cycle stasis in cells of the population, and form static tumor cells. By "contacting" is meant that the therapeutic compositions of the present invention are placed in close enough proximity to the targeted tumor such that a tumor-inhibiting effect is observed, or that the therapeutic compositions are placed in a medium, such as a nutrient medium or blood, such that the level of methionine in the medium is decreased to a level where the growth of tumor cells in the medium are inhibited. By "static" tumor cells is meant tumor cells that are growth inhibited. The method can be practiced *in vitro* or *in vivo.* The contacting of the tumor cells does not need to be direct and may be executed by connecting the nutrient medium containing the tumor cells, either *in vitro* or *in vivo*. The contacting may also be accomplished by contacting circulating blood with methioninase. Such circulating blood may or may not contain tumor cells as the tumors may be solid tumors that do not circulate. By "inhibiting tumor cell growth" is meant that administration of the compound of the invention reduces the growth of a tumor, as measured by, for example, size, by 10% to 100%, more preferably by 34% to 79%, and more preferably by 55% to 68% when compared to a tumor that has not been treated with the compound.

Methioninase is used as an antitumor reagent to slow or stop cell division by depleting methionine. This effect can be enhanced with competitive inhibitors of methionine. In addition, methioninase can be used in combination with anti-mitotic and other cell-cycle specific cytotoxic agents to increase the therapeutic effectiveness of the antimitotic or other cell-cycle specific cytotoxic agents by inducing cell cycle synchronization.

Methioninase is also used in homocysteine depletion chemotherapy to reduce the risk of and to treat cardiovascular disease. Methioninase is also used to deplete blood and tumor [¹²C] methionine prior to repletion with [¹¹C] methionine to detect and diagnose tumors in the body at ultra-high resolution.

In one embodiment, the tumor cells are first subjected to a methionine-starvation step to first reduce the level of methionine. Optionally, the methionine starvation may be accompanied by homocysteine to reduce toxicity to normal cells, thereby increasing the specificity of the therapy.

Another related aspect described herein features the use of the present methionine-depletion methods to arrest tumor cells in late S/G₂ phase of the cell cycle, followed by treatment to synchronously initiate cell-cycling and the administration of a cell cycle-specific cytotoxic agent such as an antimitotic to selectively and effectively kill tumor cells. Those of ordinary skill in the art are familiar with cell cycle-specific cytotoxic agents that are toxic to cells of particular phases of the cell cycle. The method comprises the steps of:
(a) contacting the static tumcr cells produced by the previous methionine-depletion step with a cell cycle-inducing amount of methionine to initiate cell-cycling of the static tumor cells forming cycling cells, and
(b) contacting the cycling cells with a cell cycle-specific cytotoxic agent in an amount sufficient to inhibit mitosis of the mitotic cells, thereby inhibiting tumor cell growth.

In one embodiment, the method comprises the steps of
(a) contacting the static tumor cells produced by the previous methionine-depletion step with a cell cycle-inducing amount of methionine to initiate mitosis of the static tumor cells forming mitotic cells, and
(b) contacting the mitotic cells with an anti-mitotic agent in an amount sufficient to inhibit mitosis of the mitotic cells, thereby inhibiting tumor cell growth.

The invention also features the use of methioninase to lower homocysteine levels in patients to reduce the risk of, and to treat, cardiovascular diseases.

Described herein is a method of depleting methionine by administration of methioninase for tumor diagnosis and imaging.

Also featured are therapeutic compositions for methionine depletion therapy comprising a therapeutically effective amount of substantially isolated methioninase having a specific activity of at least about 10 to about 50 units methioninase activity per milligram (mg) protein and less than about 1 to about 100 ng of endotoxin per mg protein. In the preferred embodiment, the therapeutic composition contains less than 10 nanograms endotoxin per mg protein, together with a pharmaceutically acceptable carrier.

The therapeutic compositions described herein also include an endotoxin-free methioninase. In one embodiment, the methioninase is prepared using a novel and efficient method from Pseudomonas putida grown under improved fermentation conditions. To this end, the present invention also provides a method of isolating an improved, high-methioninase-producing strain of P. putida. In another embodiment, the methioninase is produced from a recombinant host containing an expression module that encodes methioninase.

The methioninase compositions of the present invention are provided in a chemically modified form by coupling of the methioninase to polymers such as polyethylene glycol (PEG). The PEG-modified methioninase of the present invention exhibits high methionine depletion activity, an extended half-life, and low immunogenicity.

The methioninase compositions of the present invention are also provided as a recombinant protein produced using an expression vector such as a high expression vectors are herein defined. Methioninase produced using a high expression system can be readily purified using the methods described herein to obtain a methioninase composition that is endotoxin free and highly pure, having a specific activity of about 10 to about 50 units/mg methioninase.

The invention further provides vectors containing nucleic acid molecules that express unexpectedly high levels of methioninase. Such vectors, for example these that utilize the T7 RNA polymerase promoter, have been used to produce methioninase at about 1 to about 4 grams/liter with a specific activity of about 2.6 to about 5 in the crude homogenate, representing from about 10 to about 20% of the total cellular protein under appropriate incubation conditions.

The invention further provides gene therapy methods that utilize a cloned methioninase gene that can be used in place of compositions containing methioninase for the herein disclosed methods. Cells can be altered to express methioninase within a patient as a means of supplying a therapeutic amount of methioninase to a patients serum.

Other features, advantages and related embodiments of the present invention will be apparent based on the disclosures contained herein.

### Brief Description of the Drawings

Figure 1 is a compilation of 6 graphs illustrating the growth of tumor cell lines in methione-free, homocysteine-free, or methione and homocysteine-free media.
Figure 2 is a graph that illustrates the efficacy of methioninase for reducing the level of human lung tumor (H460) growth in mouse xenografts. The results of 5-FU and vincristine treatment are also illustrated.
Figure 3 is a graph that illustrates the effect of methioninase, 5-FU, and vincristine on the body weight of mice implanted with a human lung tumor. (H460)
Figure 4, 5 and 6 are graphs of the pharmacokinetics of methioninase and methionine after administration of methioninase to three human patients. The percentage of methioninase activity is measured as a relative percentage of activity of the starting preparation of methioninase. The percentage of methionine is measured as a relative percentage of the methionine concentration before administration of methioninase.
Figure 7 is an illustration of the methioninase-homocysteine metabolic cycle.
Figure 8 provides the nucleotide sequence and corresponding amino acid sequence of a methioninase encoding DNA molecule isolated from *P*. *putida*.
Figure 9 provides a diagram of representative high expressionmodules contained in vectors.
Figure 10 provides an outline of the purification steps used to obtain highly pure, endotoxin free methioninase.
Figure 11 provides an overview of typical purity and recovery yields for rMETase using the purification method herein described.
Figure 12 provides an example of the purity of rMETase produce by the present methods.
Figure 13 provides an activity profile for different rMETase formulations.
Figure 14 provides HPLC analysis data for PEG-rMETase.
Figure 15 provides HPLC analysis data for PEG-rMETase.
Figure 16 provides the pharmacokinetics of PEG-rMETase in mice.
Figure 17 provides the growth inhibition provided by rMETase on K33-1 cells in vitro.
Figure 18 provides efficacy of rMETase against KB3-1 cells in nude mice.
Figure 19 provides the toxicity of rMETase on body weight in nude mice.
Figure 20 provides the toxicity of rMETase on blood cells in nude mice with KB3-1 cells.
Figure 21 provides the toxicity of rMETase in BALB/C mice.
Figure 22 provides the toxicity of rMETase in BALB/C mice.
Figure 23 provides a toxicity evaluation of methioninase in human patients.
Figure 24 provides a pharmacokinetic evaluation of rMETase in a human patient.
Figure 25 provides data demonstrating growth inhibition of rMETase on H460 and HT29 in nude mice.
Figure 26 provides a comparison of sensitivities to rMETase between normal cells and human cancer cells *in vitro*.

The drawings are not necessarily to scale, and certain features of the invention may be exaggerated in scale and shown in schematic form in the interest of clarity and conciseness.

### Description of the Invention

### A. Definitions

"Amino acid residue" refers to an amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. Chem. 243:3552-59, 1969, and adopted at 37 C.F.R. 1.822(b)(2)), hereby incorporated by reference.

It should be noted that all amino acid residue sequences represented herein by formulae have a left-to-right orientation in the conventional direction of amino terminus to carboxy terminus. In addition, the phrase "amino acid residue" is broadly defined to include the amino acids listed in the Table of Correspondence and modified and unusual amino acids, such as those listed in 37 CFR 1.822(b) (4), and incorporated herein by reference. Furthermore, it should be noted that a dash at the beginning or end of an amino acid residue sequence indicates a peptide bond to a further sequence of one or more amino acid residues or a covalent bond to an amino-terminal group such as NH₂ or acetyl or to a carboxy-terminal group such as COOH.

"Recombinant DNA (rDNA) molecule" refers to a DNA molecule produced by operatively linking two DNA segments. Thus, a recombinant DNA molecule is a hybrid DNA molecule comprising at least two nucleotide sequences not normally found together in nature. rDNA's not having a common biological origin, i.e., evolutionarily different, are said to be "heterologous".

"Vector" refers to a rDNA molecule capable of autonomous replication in a cell and to which a DNA segment, e.g., gene or polynucleotide, can be operatively linked so as to bring about replication of the attached segment. Vectors capable of directing the expression of genes encoding for one or more polypeptides are referred to herein as "expression vectors". Particularly important vectors allow convenient expression of a methioninase protein of this invention.

### B. Methioninase as an Antitumor Agent

### I. Methionine Depletion

Methioninase is used herein as an antitumor agent in a variety of modalities for substantially depleting methionine from a tumor cell, tumor tissue or the circulation of a mammal with cancer, or for depletion of methionine where its depletion is considered desirable, as described further herein.

Whereas prior methods can reduce the levels *in vivo* of methionine to no lower than about 35 micromolar (uM), no methods are known which can safely, easily and rapidly reduce methionine levels substantially below about 10 uM. By substantially is meant at least detectably below 10 uM, preferably less that 1 uM, more preferably less than 0.1 uM, and most preferably to non-detectable levels, using conventional methionine detection methods. Methionine can be measured in aqueous solutions, including body fluids such as blood, plasma and serum, by a variety of methods. An exemplary method is reverse-phase FPLC using methionine standards.

Depletion can be conducted *in vivo,* in the circulation of a mammal, *in vitro* in cases where methionine depletion in tissue culture or other biological mediums is desired, and in ex vivo procedures where biological fluids, cells or tissues are manipulated outside the body and subsequently returned to the body of the patient mammal.

Depletion of methionine from circulation, culture media, biological fluids or cells is conducted to reduce the amount of methionine accessible to the material being treated, and therefore comprises contacting the material to be depleted with a methionine-depleting amount of methioninase according to the present invention under methionine-depleting conditions as to degrade the ambient methionine in the material being contacted.

Because tumor cells are dependent upon their nutrient medium for methionine, the depletion may be directed to the nutrient source for the cells, and not necessarily the cells themselves. Therefore, in an *in vivo* application of the present invention, the methioninase can be contacted with the nutrient medium for a population of tumor cells. In this embodiment, the medium can be blood, lymphatic fluid, spinal fluid and the like bodily fluid where methionine depletion is desired.

A methionine-depleting amount can vary widely depending upon the application, and typically depends upon the amount of methionine present in the material, the desired rate of depletion, and the tolerance of the material for exposure to methioninase. Methionine levels in a material, and therefore rates of methionine depletion from the material, can readily be monitored by a variety of chemical and biochemical methods well known in the art. Exemplary methionine-depleting amounts are described further herein, and can range from 0.001 to 100 units (U) of methioninase, preferably about 0.01 to 10 U, and more preferably about 0.1 to 5 U methioninase per milliliter (ml) of material to be treated.

Methionine-depleting conditions are buffer and temperature conditions compatible with the biological activity of the methioninase enzyme, and include moderate temperature, salt and pH conditions compatible with the enzyme. Exemplary conditions include about 4-40 degrees Centigrade (°C), ionic strength equivalent to about 0.05 to 0.2 M NaCl, and a pH of about 5 to 9, although physiological conditions are preferred.

In a preferred embodiment, the invention contemplates methioninase as an antitumor agent and use of methioninase for the manufacture of a medicament for treating tumors, and therefore comprises contacting a population of tumor cells with a therapeutically effective amount of methioninase for a time period sufficient to induce cell cycle statis in cells of the population, and form static tumor cells.

As described herein, the placement of tumor cells in cell cycle stasis is desirable for a variety of reasons, including but not limited to, selectively slowing tumor growth, killing tumor cells by maintaining the cells in stasis for such prolonged time periods as to produce cell death, and preparation of a population of tumor cells for cell cycle synchronization prior to a subsequent chemotherapeutic treatment, and the like.

The time period required to induce cell cycle stasis by contact with methioninase depends on several factors, such as the amount of methioninase contacted with the cells or the medium containing the cells, the amount of methionine, specific activity of the enzyme, temperature and other reaction conditions affecting reaction rate, and the like parameters readily controllable by the practitioner. Typical time periods are 10 minutes to about 30 days, preferably about 1 hour to 20 days, and more preferably about 1 to 10 days.

Cell cycle stasis is a condition in which the cell is not dividing nor cycling through the full cycle of events, the phases being individually referred to as G₀, G₁, G₂ and S phases. Upon methionine depletion, it is believed that the cell stops cycling in the late S/G₂ phase as evidenced by the accumulation of DNA relative to normal resting cells. Methods for identifying cell cycle stasis can be determined by a variety of histological methods, and can be evaluated by cell culture, and involve measuring DNA content of a population of cells as described in the Examples.

Tumors for which the present compounds, compositions and uses are useful include any malignant cell type such as is found in a solid tumor or a hematological tumor. Exemplary solid tumors can include, but are not limited to a tumor of an organ selected from the group consisting of pancreas, colon, cecum, stomach, brain, head, neck, ovary, kidney, larynx, sarcoma, lung, bladder, melanoma, prostate and breast. Exemplary hematological tumors include tumors of the bone marrow, T or B cell malignancies, leukemias, lymphomas, blastomas, myelomas, and the like.

In one embodiment, the contacting *in vivo* is accomplished by administering, by intravenous or intraperitoneal injection, a therapeutically effective amount of a physiologically tolerable composition containing methioninase of this invention to a patient, thereby depleting the circulating methionine source of the tumor cells present in the patient. The contacting of methioninase can also be accomplished by administering the methioninase into the tissue containing the tumor cells.

A therapeutically effective amount of a methioninase is a predetermined amount calculated to achieve the desired effect, i.e., to deplete methionine in the tumor tissue or in a patient's circulation, and thereby cause the tumor cells to stop dividing.

Thus, the dosage ranges for the administration of methioninase of the invention are those large enough to produce the desired effect in which the symptoms of tumor cell division and cell cycling are reduced. The dosage should not be so large as to cause adverse side effects, such as hyperviscosity syndromes, pulmonary edema, congestive heart failure, and the like. Generally, the dosage will vary with the age, condition, sex and extent of the disease in the patient and can be determined by one of skill in the art.

The dosage can be adjusted by the individual physician in the event of any complication.

A therapeutically effective amount of a methioninase of this invention is typically an amount such that when administered in a physiologically tolerable composition is sufficient to achieve a intravascular (plasma) or local concentration of from about 0.001 to about 100 units (U) per ml, preferably above about 0.1 U, and more preferably above 1 U methioninase per ml. Typical dosages can be administered based on body weight, and are in the range of about 5-1000 U/kilogram (kg)/day, preferably about 5-100 U/kg/day, more preferably about 10-50 U/kg/day, and more preferably about 20-50 U/kg/day.

In preferred compounds, compositions and uses, a methioninase used is substantially free of endotoxin, as discussed further herein. Particularly preferred is the use of recombinantly produced methioninase that is substantially free of endotoxin.

The methioninase can be administered parenterally by injection or by gradual infusion over time. Methioninase can be administered intravenously, intraarterially, intraperitoneally, orally, intramuscularly, subcutaneously, intracavity, transdermally, dermally, can be delivered by peristaltic means, or can be injected directly into the tissue containing the tumor cells or can be administered by a pump connected to a catheter that may contain a potential biosensor or methionine.

The therapeutic compositions containing methioninase are conventionally to be administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent; i.e., carrier, or vehicle.

The compositions are to be administered in a manner compatible with the dosage formulation, and in a therapeutically effective amount. The quantity to be administered depends on the subject to be treated, capacity of the subject's system to utilize the active ingredient, and degree of therapeutic effect desired. Precise amounts of active ingredient required to be administered depend on the judgment of the practitioner and are peculiar to each individual. However, suitable dosage ranges for systemic application are disclosed herein and depend on the route of administration. Suitable regimes for initial administration and booster shots are also contemplated and are typified by an initial administration followed by repeated doses at one or more hour intervals by a subsequent injection or other administration. Exemplary multiple administrations are described herein and are particularly preferred to maintain continuously high serum and tissue levels of methioninase and conversely low serum and tissue levels of methionine. Alternatively, continuous intravenous infusion sufficient to maintain concentrations in the blood in the ranges specified for *in vivo* therapies are contemplated.

### II. Methionine Depletion

In practicing the present invention, methionine depletion by contacting a methionine-containing material with methioninase as described earlier can be accompanied by one or more supplementary methionine depletion steps. Furthermore, the invention contemplates a variety of treatment procedures where the depletion of methionine is desirable, as described further herein.

### a. Methionine Starvation

For example, a methionine starvation step may be used to first reduce the ambient levels of methionine, wherein the starvation step involves contacting the tumor cell(s) with methionine-free nutrients for a methionine starvation time period that is conducted prior to or during the methioninase contacting step. A methionine starvation step may comprise the use *in vitro* of methionine-deficient medium, i.e., medium with low levels or no methionine, or the use *in vivo* of a methionine-free diet. Methionine-free media are well known in the tissue culture arts. An exemplary medium is Eagle's minimal essential medium (lacking methionine and choline chloride) with nonessential amino acids, such as is available from GIBCO. Methionine deficient amino acid diet foods are also commercially available, including diet TD 92077 available from Teklad, Inc.

The time period for a methionine starvation step can vary widely depending on the particular application, and is a time period sufficient for cell culture, tissue or vascular concentrations of methionine to decrease to a lower level, and stop any further substantial decrease in level. Typical time periods can be from about 6 hours to about 2 months, preferably about 1 day to 2 weeks.

Because methionine is an essential amino acid, it is to be understood that many applications of the present methods can be somewhat toxic to normal cells. However, as described herein, normal and tumor cell metabolize the methionine precursor homocysteine differently, such that homocysteine can supplement methionine deficiencies in normal cells while not rescuing methionine dependencies of tumor cells.

Thus, in a related embodiment the invention contemplates the use of a methionine-deficient nutrient (medium or diet) which may be supplemented with a methionine precursor such as homocysteine, or an analog thereof, used by normal cells to provide necessary nutritional supplements. Homocysteine can be added to nutrient medium at a concentration of about 5 to about 200 micromolar (uM), preferably about 10 to about 100 uM.

Preferred methionine precursors useful in this embodiment include L-homocysteine-thiolactone, homocysteine, and 4-methylthio-2-oxobutanoic acid.

Thus, in one embodiment, the invention comprises the step cf feeding a mammal with a methionine deficient diet for a time period to deplete the intravascular concentrations of methionine. The diet may optionally contain methionine precursors as a methionine supplement. Alternatively, the methionine precursor may be administered by injection or other routes as is well known. Preferred daily dosages of homocysteine as a dietary supplement are about 5 to about 1000 mg homocysteine per kilogram mammal body weight per day.

The routes of administration of the homocysteine are typically the same as the methioninase to be added, and depend on the target tissue for delivery of the supplement.

### b. Competitive Inhibitors of Methionine Utilizing Enzymes

Following the observations described in the Examples, it has been further discovered that competitive inhibitors of methionine-utilizing enzymes are useful to synergistically increase the effectiveness of methionine depletion methods described herein. Thus the invention contemplates the use of a methionine starvation step that further comprises contacting the tumor cells with an amount of a competitive inhibitor of methionine for a time period sufficient to inhibit methionine metabolism by a methionine-utilizing enzyme. Methionine-utilizing enzymes useful for directing competitive inhibitors include 5-adenosyl methionine decarboxylase, methionine t-RNA synthase and methionine adenosyltransferase.

Preferably, the time period required to inhibit methionine is the duration of the methionine starvation time period itself.

Competitive inhibitors of methionine compete with methionine as a substrate for methionine-utilizing enzymes, and therefor act to inhibit any normal metabolic effect that endogenous methionine might produce by direct competition, i.e., inhibits methionine metabolism. Where the cell requires methionine and the attendant metabolism of methionine, the competitive inhibitor acts to reduce the metabolism of methionine, necessitating higher methionine concentrations to yield the same effect observed where no inhibitor is present.

A competitive inhibitor of methionine can be any methionine derivative that functions as a classic competitive inhibitor. Typical competitive inhibitors include alkyl derivatives of methionine (i.e., alkylthionines), as where the methyl group of methionine is replaced with an ethyl group (ethionine), a propyl group (propthionine), a butyl group (buthionine), or a pentyl group (penthionine).

Also contemplated as useful methionine competitive inhibitors are cycloleucine and halogenated methionines. A typical halogenated methionine is selected from the group consisting of fluoromethionine, chloromethionine, bromomethionine and iodomethionine. Thus a contemplated competitive inhibitor of methionine is selected from the group consisting of alkylthionine, cycloleucine and a halogenated methionine, wherein said alkylthionine is not methionine.

An amount of a competitive inhibitor of methionine effective to competitively inhibit methionine is an amount to produce a reduction in the effective concentration of methionine. This amount is typically a molar excess relative to the methionine present, as shown in the Examples. Typically, this amount of inhibitor is in the range of a 10 to 1000 fold molar excess relative to the methionine concentration in the medium where methionine is to be competed, preferably at least a 20 fold molar excess, and more preferably at least a 50 fold molar excess. Where the inhibitor is to be used *in vivo,* the dosage is typically about 5 - 30 mg per kg of animal body weight, preferably about 25 mg/kg, as shown herein.

The amount of inhibitor required for effectiveness may vary depending upon the amount of endogenous methionine present at the time the inhibitor is administered. This relationship between concentration of methionine and effective concentration of inhibitor is demonstrated by the dosage titration results shown in the Examples, and is defined by classic reaction rate thecry for competitive inhibitors. Typical amounts of inhibitor are from about 10 uM to about 1 mM.

Furthermore, the amount of effective inhibitor can be predetermined by the *in vitro* histoculture methods described herein, to first establish the effective conditions in a particular tumor tissue for methionine depletion by any of the methods described herein, followed by a determination of the effective concentration of the inhibitor.

The inhibition of methionine metabolism by use of a competitive inhibitor of methionine can be monitored by a variety of indicators, including the indicators described in the Examples. These indicators include histoculture of a tissue to measure DNA content as an indicator of cell cycle arrest, and increased inhibition of growth of tumor tissue in a mouse fed a methionine-free diet. Other indicators will be apparent to one skilled in the art.

### c. Synergistic Effect of Using Multiple Methionine

Depletion Methods - Anti-Methionine Chemotherapy One embodiment, described herein contemplates the use of multiple methods for methionine depletion, generally referred to as anti-methionine chemotherapy, to take advantage of the synergy that occurs when two or more different methods for methionine depletion are used.

Methionine depletion methods described herein include (1) methionine starvation using methionine-free medium (*in vitro*) or diet (*in vivo*), (2) exposure to methioninase to enzymatically deplete endogenous methionine levels, (3) competitive inhibitors of methionine to reduce the effective concentration of endogenous methionine, particularly in combination with methods (1) and (2) above, where methionine levels are already reduced, and (4) use of methionine precursors, such as homocysteine described herein, to increase the selectivity of any of the other three methionine depletion methods for tumor cells.

Thus, anti-methionine chemotherapy methods described herein for methionine depletion can be conducted by any of a variety of combinations of the above identified methods, including (1) plus (2), (1) plus (3), (2) plus (3), (1) plus (2) plus (3), and any of these four combinations plus (4).

Particularly preferred for maximum depletion of methionine and its resultant metabolites is the use of methionine-free diet but containing methionine precursors, plus the use of a competitive inhibitor of methionine and methioninase.

### III. Enhancement of Anti-Mitotic Tumor Therapy

In another embodiment, the use of methionine-depletion methods described herein in methods for increasing (enhancing) the potency and selectivity of conventional chemotherapies, and preferably the selectivity of antimitotic drugs used for cancer therapy is contemplated.

The key purpose for use of methionine-depletion steps in combination with conventional antimitotic therapies is to take advantage of the fact that methionine depletion selectively and specifically stops tumor cell proliferation prior to mitosis, such that upon repleting the cell, tissue, medium or vasculature with methionine, the static cells synchronously begin cell cycling, into mitosis rendering the population of cells to be uniformly proliferating and susceptible to the effects of cell-cycle specific chemotherapy. To cause the tumor cells to enter cell stasis, any cell cycle cytotoxic agent may be used. Such agents are well known to those of ordinary skill in the art. in a preferred embodiment, the cell-cycle cytotoxic agent is an anti-mitotic agent.

Thus, in a preferred embodiment, the present invention contemplates use of methioninase for the manufacture of a medicament for antitumor chemotherapy, said chemotherapy comprising the methionine-depleting step described herein to form static tumor cells followed by the additional steps of:
(a) contacting the static tumor cells with a cell cycle-inducing amount of methionine to initiate mitosis of the static tumor cells, thereby forming mitotic cells, and
(b) contacting the mitotic cells with an anti-mitotic agent in an amount sufficient to inhibit mitosis of the mitotic cells, thereby inhibiting tumor cell growth.

Methionine, methionine salts and functional equivalents thereof that function to initiate cell cycling of a static cell are referred to as cell cycle inducing agents, and can be used together or alternatively as the reagent for initiating cell cycling and mitosis in one or more static cells in the methionine-depleted tumor cell population.

A cell-cycle inducing amount of a cell cycle-inducing agent is an amount that initiates cell cycling in at least a few (10%), preferably a majority, and more preferably at least 90% of the static cells in the methionine-depleted tumor cell population. The initiation and extent of cell cycling can readily be measured by use of histological or metabolic markers. For methionine, a cell-cycle inducing amount is typically in the range of about 1 micromolar (uM) to about 2.5 millimolar (mM), and preferably about 10 to 250 uM. S-phase-specific drugs can similarly be used to attack any tumor cells still in S-phase when methionine is repleted.

Routes of contacting (administration) of the cell cycle inducing agent with the static tumor cells can vary but typically will be the same routes as used for administering methioninase or competitive inhibitors as described herein.

The timing and time periods for contacting the static tumor cells with a cell-cycle inducing agent can vary depending on the tumor, and other considerations that can be determined empirically, such as by the histoculture methods described herein. The cycle-inducing agent can be added prior to or substantially simultaneously with the antimitotic agent. It may be advantageous to add the inducer prior to the antimitotic agent where it has been determined that the particular antimitotic is fast acting, and cell cycle induction is slow. These parameters may depend upon the particular tumor type, tissue location, choice of antimitotic and the like. Furthermore, optimization of variables such as timing, dosage and drug choice can be readily carried out by a variety of methods prior to *in vivo* administration of the therapeutic methods. A preferred optimization procedure is to use the exemplary histoculture assay system described herein.

The timing for contacting an anti-mitotic agent depends upon the induction of cell cycling of the static tumor cells. Typically, anti-mitotic agents can be contacted with the mitotic cells at any time during cycling, and as soon as the cells became mitotic. For seme tumor cell populations, this event may occur rapidly after addition of the inducer agent, allowing simultaneous or near simultaneous addition of both inducer and anti-mitotic agent to the static tumor cells. Alternatively, the anti-mitotic may be added about 1 hour to 30 days after inducer, but preferably within about one or two days of induction.

The anti-mitotic agent or other cell-cycle specific agent used herein can be any agent exhibiting cell toxicity based mechanisms directed at cell proliferation, mitosis or cell cycling. Thus anti-mitotic agents can include any of a variety of compounds which are anti-metabolites or which otherwise exhibit toxicity for dividing (mitotic) cells. Preferred clinical pharmacology of a anti-mitotic agent for use in the present methods is inhibition of cell mitotic activity, inhibition of nucleic acid synthesis, alkylating agents, antibiotics, alkaloids, and the like antitumor agents. Insofar as the field of pharmacology is constantly advancing, it is to be understood that the invention is to not be limited to presently known anti-mitotic and other cell-cycle specific agents, but rather to include the use of equivalents of known compounds, newly discovered or developed compounds and the like agents exhibiting the requisite activity.

Exemplary alkylating agents include cyclophosphamide (CTX; cytoxan), chlorambucil (CHL; leukeran), cisplatin (CisP; platinol) busulfan (myleran), melphalan, carmustine (BCNU), streptozotocin, triethylenemelamine (TEM), mitomycin C, and the like alkylating agents.

Exemplary anti-metabolites include methotrexate (MTX), etoposide (VP16; vepesid) 6-mercaptopurine (6MP), 6-thiocguanine (6TG), cytarabine (Ara-C), 5-fluorouracil (5FU), dacarbazine (DTIC), and the like anti-metabolites.

Exemplary antibiotics include actinomycin D, doxorubicin (DXR; adriamycin), daunorubicin (daunomycin), bleomycin, mithramycin and the like antibiotics.

Exemplary alkaloids include vinca alkaloids such as vincristine (VCR), vinblastine, and the like.

Other antitumor agents include taxol and taxol derivatives, the cytostatic agents glucocorticoids such as dexamethasone (DEX; decadron) and corticosteroids such as prednisone, nucleoside enzyme inhibitors such as hydroxyurea, amino acid depleting enzymes such as asparaginase, and the like diverse antitumor agents.

The synthesis and formulation of the above anti-mitotic (cytotoxic) agents is well known, is described in a variety of sources, and therefore will not be repeated here. Exemplary sources for synthesis and formulations of the above agents include Physician's Desk Reference, Barnhart, eds., Medical Economics Company, Inc., Oradell, N.J., 1992; and Merck Index, 11th Edition, Merck & Co., 1989.

The use of cytotoxic agents described above in chemotherapeutic regimens is generally well characterized in the cancer therapy arts, and their use herein falls under the same considerations for monitoring tolerance and effectiveness and for controlling administration routes and dosages, with some adjustments. For example, the actual dosages of the cytotoxic agents may vary depending upon the patient's cultured cell response determined by using the present histoculture methods. Generally, the dosage will be reduced compared to the amount used in the absence of synchronized cell cycling, as shown by the present disclosures.

Typical dosages of an effective cytotoxic agent can be in the ranges recommended by the manufacturer, and where indicated by *in vitro* responses or responses in animal models, can be reduced by up to about one order of magnitude concentration or amount. Thus, the actual dosage will depend upon the judgment of the physician, the condition of the patient, and the effectiveness of the therapeutic method based on the *in vitro* responsiveness of the primary cultured malignant cells or histocultured tissue sample, or the responses observed in the appropriate animal models.

### IV. Gene Therapy

In another embodiment, the use of a rDNA for expression of methioninase within the tissue or lymphocytes surrounding the tissue to be treated according to the invention is described. To that end, it is contemplated that the methioninase enzyme is presented for the contacting step by the expression of a regulatable methioninase expressing gene, thereby producing the methioninase gene product.

As described earlier, there are a large variety of suitable expression vectors presently known for expressing methioninase which can be used. In one embodiment, the expression of the methioninase gene is regulatable. Thus an expression vector has a second nucleotide sequence operably linked to the first methioninase encoding sequence which defines a means for regulating the expression of the methioninase gene. An exemplary means is an inducible promoter, the preferred being promoters that are tumor specific. Expression modules in which the expression of the methioninase gene is controlled by a tumor specific promoter are particularly useful for gene therapy as herein described.

One class of inducible promoters are responsive to a component that can be added to the culture medium, or readily penetrate into a host cell containing the gene for expressing the methioninase gene. Another class are tumor specific promoters as described above, for example the carcinoembryonic antigen (CEA) promoter.

Thus, also described is a methionine depleting method wherein the contacting of methioninase with a population of tumor cells comprises the steps of:
(i) introducing an expression vector into the tumor cells or into tumer infiltrating lymphocytes or their bone marrow precursors *in vivo* or *ex vivo,* wherein the expression vector has a nucleotide sequence that encodes methioninase and is capable of regulated expression of methioninase, and has a nucleotide sequence that provides a means for regulating the expression of methioninase, to form methioninase gene transfected cells;
(ii) contacting the tumor cells *in vivo* with the methioninese gene transfected cells; and
(iii) expressing the methioninase-encoding nucleotide sequence in the transfected cells, thereby producing methioninase in the transfected cells and thereby contacting tumor cells with the produced methioninase. By "introducing" is meant any method known by those of ordinary skill in the art to insert the expression vector into the target cells in a manner wherein the gene contained within the expression vector may be expressed by the target cell. The introduction can be performed *ex vivo* or *in vivo*. Such "introducing" may be accomplished by, for example, transfection, transformation, or viral infection.

Transfection of tumor cells, either adherent cells or cells in suspension or *in vivo*, can be accomplished by a variety of known transfection methods. Thereafter, the transfected cell (if *ex vivo* tranfection is used) is reintroduced into the host and allowed to locate in the native, relevant, vicinity of tissue to be treated, thereby contacting the transfected cells with the tumor cells to be treated. Introduction of methioninase to transfected cells can be accomplished by a variety of means, but generally requires the presence of a selectable marker.

In one embodiments, the means for regulating the expression of the gene is an inducible promoter that is responsive to a reagent that can be added to the medium. Exemplary is the metallothionine promoter. Alternatively, the inducible promoter can be a tumor specific promoter. Such a promoter acts to target methioninase expression to the tumor cell.

The cells to be transfected can be a sample of the tumor cells, or normal immune cells such as tumor infiltrating cells, such as tumor infiltrating lymphocytes or bone marrow precursors cf the bone marrow, such as hematopoietic stem cells or progenitor cells. The cells can be either *in vivo* or *ex vivo*.

Example 9 describes the isolation of a nucleic acid molecule encoding methioninase. The Example further describes the generation of expression vectors for use in batch fermentation for producing methioninase. A skilled artisan can readily use the cloned methioninase gene, such as that provided in pAC-1, to produce expression cassette suitable for use in the above-described gene therapy.

### C. Therapeutic Compositions

Therapeutic compositions comprising a therapeutically effective amount of substantially isolated methioninase preferably recombinantly produced, together with a pharmaceutically acceptable carrier are described.

L-Methioninase (L-methionine-alpha-deamino-gammamercaptomethane-lyase or methioninase) is an enzyme that degrades methionine by deamination and dethiomethylation. Methicninase activity can be measured at least by measuring the amount of alpha-ketobutyrate formed upon cleavage of methionine. One unit (U) of methioninase is defined as an amount of enzyme that produces 1 micromole of alpha-ketobutyrate per minute from methionine under the standard assay conditions described by Ito et al., J. Biochem., 79:1263-1272, 1976; and Soda, Analyt. Biochem. 25:228-235, 1968.

Methioninase can be prepared from a variety of sources, including being isolated directly from bacteria cultures, or being expressed from a recombinant DNA molecule encoding a methioninase protein, such as described in Examples 9 and 12.

Bacterial sources of methioninase include Pseudomonas putida, Pseudomonas ovalis, and Aeromonas sp. and any other potential sources of methioninase. P. putida strains are commercially available from the ATCC and have accession numbers ATCC 8209 and ATCC 7955, respectively. The other bacteria are generally available from the academic research community. Purification of methioninase has been described by a variety of methods. See, for example, Kreis et al., Cancer Res., 33:1862-1865, 1973; Tanaka et al., FEBS Letters 66:307-311, 1976; Ito et al., J. Biochem. 79:1263-1272, 1976; Nakayama et al., Agric. Biol. Chem. 48:2367-2369, 1984; and Soda, Analyt. Biochem. 25:228-235, 1968. However, none of these methods results in a highly pure, endotoxin free methioninase. The present invention provide two methods that have been used to purify methioninase so that it is substantially endotoxin free.

A preferred methioninase has a specific activity of about 10 to about 50 units (U) per mg protein. Typical preparations of purified methioninase are described herein having a specific activity of about 10 to about 50 U/mg. In Example 12, methioninase prepared using the expression vector pAC-1 had a specific activity of about 20.1 U/mg.

A preferred methioninase is preferably substantially isolated. By substantially isolated is meant that the enzyme is at least 50% pure by weight, preferably at least 90% pure, and more preferably at least 99% pure, or essentially homogeneous. A preferred protein is essentially homogeneous when analyzed on electrophoretic media such as polyacrylamide gel electrophoresis (PAGE). Homogeneous on PAGE means only a single detectable band.

preferred methioninase is substantially free of endotoxins, such as bacterial lipopolysaccharides, due to the undesirable side effects associated with endotoxins when physiologically contacted in a mammal, as by i.v. or i.p. administration. By substantially free is meant less than about 10 nanograms (ng) endotoxin per milligram (mg) methioninase protein, preferably less than 1 ng endotoxin per mg methioninase, and more preferably less than 0.1 ng endotoxin per mg methioninase. The assay of endotoxin is well known in the therapeutic arts, and can be conducted by any of variety of methods. A preferred method is described in the Examples.

A preferred methioninase is heat stable, so as to increase its shelf life and effectiveness when utilized under conditions of elevated temperatures, such as *in vivo* in an animal body. By heat stable is meant that the enzyme can be exposed to 60 degrees Centigrade (60°C) for 10 minutes and retain 80%, preferably retain 90%, and more preferably retain 95% of its specific activity.

A preferred methioninase exhibits-a serum half-life of at least 5 hours, preferably 6 hours, and more preferably at least 7 hours.

Particularly preferred is methioninase prepared from P. putida, or using an expression vector containing a DNA molecule isolated from P. putida that encodes methioninase. P. putida methioninase exhibits an apparent molecular weight when analyzed on PAGE-SDS under denaturing conditions of about 43 kilodaltons. A preferred method of purifying methioninase is described in the Examples.

Thus a therapeutic composition comprises a physiologically tolerable carrier together with substantially isolated methioninase, dissolved or dispersed therein as an active ingredient. In a preferred embodiment, the therapeutic composition is not immunogenic when administered to a human patient for therapeutic purposes.

One embodiment of the present invention features a methioninase that is chemically modified comprising methioninase conjugated to a polymer. Preferably, the methioninase is substantially isolated and substantially endotoxin free. By "chemically modified" is meant any form of methioninase that is changed to a form that is different than the methioninase purified from nature. Preferably, the methioninase is chemically modified by linking the methioninase to a polymer such as polyethylene glycol.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal or human without the production of undesirable physiological effects such as nausea, dizziness, gastric upset and the like.

The preparation of a pharmacological composition that contains active ingredients dissolved or dispersed therein is well understood in the art. Typically such compositions are prepared as sterile injectables either as liquid solutions or suspensions, aqueous or non-aqueous, however, solid forms suitable for solution, or suspensions, in liquid prior to use can also be prepared. The preparation can also be emulsified. Particularly preferred are phospholipid and liposome compositions as described herein. In addition, a therapeutic amount of methioninase can be present in a ointment or on a diffusible patch, such as a bandage, as to afford local delivery of the agent.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, or the like and combinations thereof. In addition, if desired, the composition can contain minor amounts of auxiliary substances such as wetting or emulsifying agents, pH buffering agents and the like which enhance the effectiveness of the active ingredient.

The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Physiologically tolerable carriers are well known in the art. Exemplary of liquid carriers are sterile aqueous solutions that contain no materials in addition to the active ingredients and water, or contain a buffer such as sodium phosphate at - physiological pH value, physiological saline or both, such as phosphate-buffered saline. Still further, aqueous carriers can contain more than one buffer salt, as well as salts such as sodium and potassium chlorides, dextrose, propylene glycol, polyethylene glycol and other solutes.

Liquid compositions can also contain liquid phases in addition to and to the exclusion of water, as described herein. Exemplary of such additional liquid phases are glycerin, vegetable oils such as cottonseed oil, organic esters such as ethyl oleate, and water-oil emulsions, particularly the liposome compositions described earlier.

A therapeutic composition contains an effective amount of methioninase, typically an amount of at least 0.1 weight percent of active protein per weight of total therapeutic composition, and preferably is at least about 25 weight percent. A weight percent is a ratio by weight of methioninase protein to total composition. Thus, for example, 0.1 weight percent is 0.1 grams of methioninase per 100 grams of total composition.

Insofar as a methioninase composition can be used *in vivo* intravascularly, it is contemplated in one embodiment to formulate a therapeutic composition for controlled delivery of the methioninase, and optionally to shield the methioninase protein from degradation and other phenomenon which would reduce the serum half-life of therapeutically administered methioninase.

Thus, in one embodiment, the invention contemplates therapeutic compositions containing delivery vehicles such as polymers, polymeric vehicles, particulates, latexes, ccacervates, ion-exchange resins, liposomes, enteric coatings, mediators, bioadhesives, microcapsules, hydrogels, and the like vehicles. Exemplary drug delivery vehicles including liposomes are described at least by Tarcha in "Polymers For Controlled Drug Delivery", CRC Press, Boca Raton, 1990.

The present invention further provides detailed methods for purifying methioninase that provide superior yields and purity when compared to other known methods. The preferred method for purifying recombinant methioninase, particularly that produced using a transformed host containing a high expression module encoding methioninase, comprises the steps of:
a) heating an extract of a transformed cell that contains methioninase in aqueous buffers from about 40-60°C for about 1-10 min., preferably 50°C for 1 min.;
b) centrifugation of the heated extract from about 10k to 20k rpm in a GS-3 rotor (Sorvall, Du Pont) for about 15 min. to 1 hour, preferably at about 13K rpm for about 30 min. at 4°C;
c) ultrafiltration of the supernatant using a filter of about 50K to 100K pore size, preferably a Millipore Pre/Scale:TFF PLHK 100 K 2.5 ft² cartridge using a 10mM potassium phosphate buffer (pH8.3);
d) DEAE ion exchange chromatography in low ionic strength (from about 10-50mM) KCl in a 10-20 mM potassium phosphate buffer at about pH 7.0-7.6, and collecting fractions containing methioninase eluted in a 40-200mM KCl gradient, preferably using DEAE-Sepharose FF column;
e) a second DEAE ion exchange chromatography in medium ionic strength (50-100mM) KCl in a 10-20 mM potassium phosphate buffer at about pH 8.0-8.6, and collecting fractions containing methioninase eluted in a phosphate buffer (pH 8.3) eluted in 100-200 mM KCl, preferably using DEAE-Sepharose FF column; and
f) contacting said fractions collected in step (e) with a chromatography medium capable of absorbing endotoxin, and collecting the eluant, thereby removing endotoxin from said eluant to form endotoxin-free methioninase having at least 20 units methioninase activity per milligram protein and from 1-100 ng of endotoxin per mg protein, preferably using an Acticlean® Etox column.

The cell extract is preferably prepared from a host cell that has been altered to express high levels of recombinant methioninase (from about 5-75% of total cellular protein) Alternatively, an organism that naturally expresses methioninase can be used as the starting material. For bacterial cell extracts, the extracts are generally prepared by first harvesting and washing bacterial cell cultures to form a cell paste/pellet, depending upon whether harvesting is by centrifugation or by hollow fiber filtration, which methods are generally well known.

The cells are then disrupted using conventional means. Preferably the cells are disrupted using a homogenizer, such as a cavitator-type homogenizer, for example, a Microfluidics Corp. Model #HC8000.

The resulting suspension is heated to precipitate selective proteins and other insoluble materials. Typical heating conditions are from about 45-60°C for 1-10 minutes. Preferred is a heating step of 50°C for 1 minute.

The heated extract is centrifuged to remove debris, and the supernatant is filtered and applied to DEAE ion-exchange chromatography medium in two steps as described above. Preferred adsorption and elution conditions are described in the Examples. Any of a variety of DEAE ion exchange column chromatography media can be used in these steps, and the choice of media is not to be construed as limiting. Commercial sources include Pharmacia Fine Chemicals, BioRad, and Sigma.

Thereafter, endotoxin is removed to produce a protein having acceptable levels of endotoxin as recited earlier. The endotoxin removal step can be carried out in any of a variety of means, as are well known, and typically involve contacting the protein in solution with a chromatography medium capable of adsorbing endotoxin, and yielding a chromatography medium eluant which contains endotoxin-free protein. The preferred commercial reagent for use in removing endotoxin is Acticlean® Etox.

### D. Chemically Modified Methioninase

Methioninase may be conjugated to a polymer with the purpose of extending its half-life and decreasing its immunogenicity or antigenicity.

There is the possibility of an allergic response in oversensitive individuals. On the other hand, anti-methioninase antibodies may shorten the half - life of methioninase.

Various approaches have been taken in attempts to solve the problem of antigenicity of polypeptides and proteins. Coupling of polymers such as polyethylene glycol to proteins is one of the approaches to reducing protein antigenicity. The present invention relates to chemical modification of methioninase produced by a novel and effective method in which purified methioninase is coupled with polyethylene glycol (PEG) under conditions which maintain the activity of the enzyme. The PEG-methioninase of this invention has an extended half life and no apparent immunogenicity.

The novel methioninase conjugated to polyethylene glycol ("PEG-methioninase" or "PEGylated methioninase") exhibits a high methionine depletion activity, an extended half-life, and low immunogenicity. Thus PEG-methioninase provides a novel means of inhibiting tumor growth while maintaining stability, increasing its serum half-life, decreasing immunogenicity and decreasing toxicity.

The present invention of PEG - methioninase which is stable, effective in methionine depletion with a long half - life and is non - immunogenic, may be utilized as a safe and effective anti - tumor agent for multiple dosing. PEG - methioninase may be stored in a liquid formulation of 0.12 M sodium chloride in 10 mM sodium phosphate (pH 7.2) at - 70°C, 4°C and at room temperature without loss of activity.

Importantly, the PEG - methioninase of this invention is non - immunogenic which renders it particularly desirable for the treatment of cancer patients who are sensitive to methioninase, and require repeated dosing. Those of ordinary skill in the art will recognize that as with methioninase, the PEG-methioninase may be provided in a variety of formulations.

A preferred formulation of the purified endotoxin - free methioninase is in 0.12 M sodium chloride in 10 mM sodium phosphate buffer (pH 7.2) at a concentration between 0.06 M and 0.2 M. The activity is approximately 10 units/mg.

The PEGylated methioninase may be tested by means well known to those of ordinary skill in the art. For example, *in vivo* testing may be used to determine the pharmacology, safety and functional characteristics of PEG- methioninase produced by the method cf the invention.

Such tests may include determination of acute toxicity, pharmacokinetics of PEG - methioninase, depletion of methionine in serum and evaluation of immunogenicity of PEG - methioninase.

Purified endotoxin-free PEG - methioninase is injected into the tail-vein of mice and the blood samples are collected every two hours. The levels of methioninase are measured by the activity assay. The levels of methionine are measured with HPLC after methionine derivitization.

Acute toxicity studies may be performed in any suitable experimental animals such as those described in Examples 5 and 15. In mice, 10 - 100 units / 1 - 10 mg of PEG - methioninase are injected into the tail-vein. Vital signs and visual observations are recorded. The blood samples are collected before the injection and every two hours after injection. Both the activity of methioninase and the levels of methionine are measured. The functions of both kidney and liver function are also measured. The presence or absence of toxic effects on tissues such as lung, kidney, liver and brain are determined by standard techniques. The blood and bone marrow are analyzed as well.

In another embodiment, the methioninase is conjugated to a polymer resulting in a substantially nonimmunogenic composition and also resulting in an increase in the half-life of methioninase activity *in vivo*.

In one embodiment the methioninase is chemically modified by conjugation to a polymer.

In another embodiment the methioninase is chemically modified by conjugation to a polyalkylene oxide. Examples of polyalkylene oxides include, but are not limited to, polyethylene oxide, polypropylene oxide, copolymers of ethylene oxide, and copolymers of propylene oxide.

In a preferred embodiment the methioninase is chemically modified by conjugation to polyethylene gylcol.

In another preferred embodiment the methioninase conjugated to polyethylene glycol is substantially free of endotoxin.

The invention also provides a pharmaceutical composition comprising a therapeutically effective amount of methioninase conjugated to a polymer. The polymer may be a polyalkylene oxide, for example, but not limited to, polyethylene oxide, polypropylene oxide, copolymers of ethylene oxide, and copolymers of propylene oxide.

In a preferred embodiment is provided a pharmaceutical composition comprising a therapeutically effective amount of methioninase conjugated to polyethylene glycol. In another preferred embodiment the pharmaceutical composition comprises a therapeutically effective amount of methioninase that is substantially free of endotoxin and is conjugated to polyethylene glycol. Such composition can be prepared from material isolated from organisms that naturally produce methioninase or preferably, from a host that has been altered to express methioninase.

Also provided is a method for producing endotoxin-free methioninase which is conjugated to a polymer by coupling endotoxin-free methioninase which is conjugated to a polymer by coupling endotoxin-free methioninase with a polymer to form a substantially nonimmunogenic chemically modified endotoxin free methioninase.

In a preferred embodiment the method includes coupling the methioninase to polyethylene glycol.

In a further preferred embodiment the coupling is performed by reacting endotoxin-free methioninase with methoxy polyethylene glycol succinimidyl carbonate.

Also provided is a method of treating a patient having a tumor comprising administering a therapeutically effective amount of methioninase.

In a preferred embodiment the methioninase is substantially free of endotoxin.

In a further preferred embodiment the methioninase is conjugated to a polymer.

In a further embodiment the polymer is a polyalkylene oxide.

In a further preferred embodiment the methioninase is conjugated to polyethylene glycol.

### E. Formulations of recombinant methioninase

The present invention further provides methioninase in lyophilized or crystalline form. In detail, it has been observed that methioninase can readily be lyophilized or crystallized using art known methods. The resulting preparation of methioninase, crystallized or lyophilized forms, were found to be highly stable, readily hydratable, and remained highly active following rehydration.

A variety of art known methods can be used to obtain methioninase in crystallized or lyophilized form. In the examples, lyophilization and crystallization of methioninase were performed using a Verdis, Freeze mobile 24, at 100 millibar, -80°C for 72 hours. A skilled artisan can readily adapt other art known procedures for use in producing lyophilized or crystallized forms of methioninase.

### F DNA Segments and Vectors

### I. Methioninase-Coding DNA Molecules

It has been found that by operably linking an isolated DNA molecule encoding methioninase to a promoter, particularly an RNA polymerase promoter such as the T7 RNA polymerase promoter, recombinant methioninase can be expressed at levels from about 5-75% of total cellular protein, when introduced into an appropriate host cell. Accordingly, the invention provides high-level expression modules that express high levels of recombinant methioninase when introduced into a host under appropriate conditions.

As used herein, a high-level expression module, or an expression module of the present invention, refers to a nucleic acid molecule that contains one or more expression control elements that direct the transcription and translation of an operably linked nucleotide sequence that encodes methioninase. The expression module can be an isolated nucleic acid molecule or can be present in a vector (described below).

The expression modules of the present invention contain control elements that direct the production of recombinant methioninase such that the recombinant methioninase produced represents from about 5-75% of total cellular protein, preferably more than 10% of total cellular protein. The preferred expression control elements are RNA polymerase promoters, the most preferred being the T7 RNA polymerase promoter. Other examples of RNA polymerase promoters include, but are not limited to, the Tac and Trc promoters.

A promoter is an expression control element formed by a DNA sequence that permits binding of RNA polymerase and transcription to occur. Promoter sequences compatible with a particular host system are known in the art and are typically provided in a plasmid vector containing one or more convenient restriction sites. Typical of such plasmids vectors are those containing the T7 RNA polymerase promoter, pT7 and pET that are available from a variety of sources such as commercial suppliers and the American Type Culture Collection.

The expression modules of the present invention further comprise a nucleic acid sequence that encodes methioninase. As used herein, a nucleic acid sequence is said to encode methioninase when the transcription and translation of the nucleic acid molecule comprising the sequence results in the production of a protein having methioninase activity.

L-Methioninase (L-methionine-alpha-deamino-gammamercaptomethane-lyase or methioninase) is an enzyme that degrades methionine by deamination and dethiomethylation. Methioninase activity can be measured at least by measuring the amount of alpha-ketobutyrate formed upon cleavage of methionine. One unit (U) of methioninase is defined as an amount of enzyme that produces 1 micromole of alpha-ketobutyrate per minute from methionine under the standard assay conditions described by Ito *et al*., J. Biochem., 79:1263-1272, 1976; and Soda, Analyt. Biochem. 25:228-235, 1968.

The methioninase-encoding nucleic acid sequence can comprise an unaltered sequence obtained from an organism that naturally produces recombinant methioninase, or can comprise a sequence obtained from an organism that naturally produces methioninase that has been altered to contain one or more nucleic acid or amino acid substitutions, deletions or additions.

The methioninase-encoding nucleic acid molecule, whether altered or unaltered, can be derived from any organism that naturally produces methioninase. The preferred source of the methioninase-encoding nucleic acid molecule is *Pseudomonas putida*. Example 9 discloses the isolation and sequencing of a methioninase-encoding nucleic acid molecule from *P. putida*. Other preferred sources for a methioninase-encoding nucleic acid molecule include, but are not limited to, *Trichomonas vaginalis, Nippostrongylus brasiliensis,* and *Fusobacterium sp.*

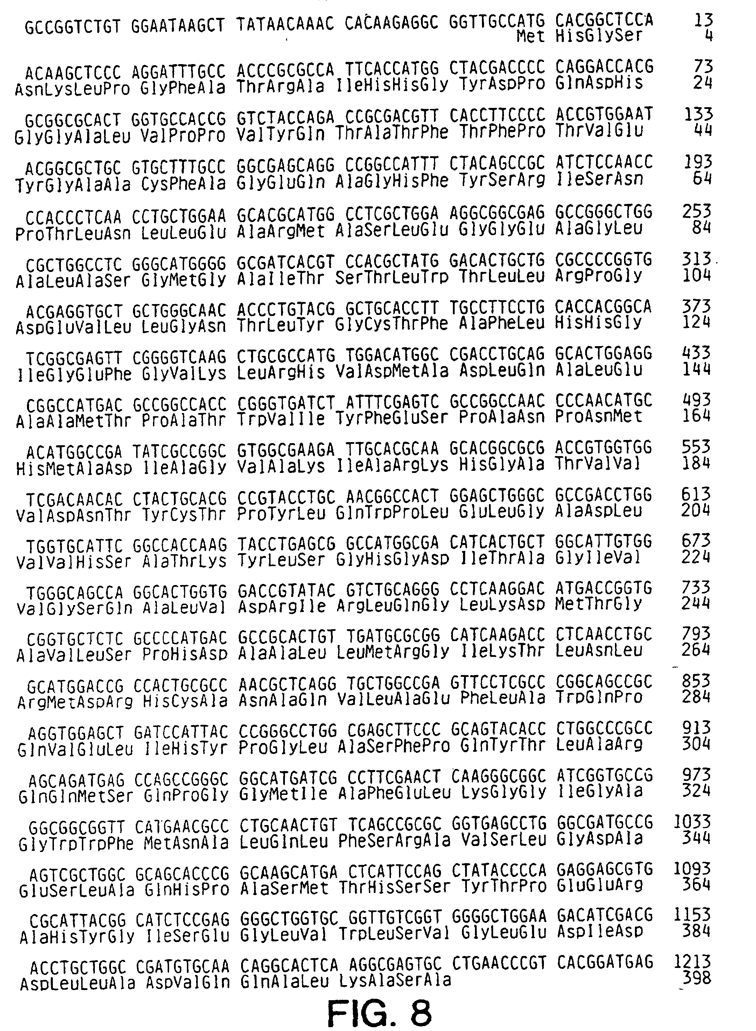

The complete coding sequence for methioninase can be obtained from a variety of sources, especially those recited above, using a variety of methods. The isolation of methioninase-encoding nucleic acid molecules from an organism other than *P. putida* is greatly facilitated by the amino acid and nucleic acid sequences provided in Figure 8.

Specifically, a skilled artisan can readily use the nucleic acid sequence provided in Figure 8 to prepare pairs of oligonucleotide primers for use in a polymerase chain reaction (PCR) to selectively amplify a methioninase-encoding nucleic acid molecule from methionine expressing organisms. The preferred PCR primer pairs based on the sequence provided in Figure 8 are: 
5'-GCCGGTCTGTGGAATAAGCT-3' (Sense)

5'-CCAGGGTCGACTCCAGCGCC-3' (Antisense)
 A preferred PCR denature/anneal/extend cycle for using the above PCR primers is as follows: first denaturation at 95°C for 10 minutes, then 5 cycles of denaturation at 94°C for 30 seconds, annealing at 60°C for 30 seconds, and extension at 72°C for 2 minutes; then 25 cycles of denaturation at 94°C for 30 seconds, 60°C for 30 seconds, then extension at 72°C for 1.5 minutes; then final extension at 72°C for 10 minutes. The PCR amplified products are two bands of which the 1365 bp band was collected, and purified as the insert ONCase-1 DNA.

Alternatively, a fragment of the nucleotide sequence Figure 8 can be used as a probe to isolate DNA encoding methioninase from organisms other than *Pseudomonas putida* using art-known methods. Oligomers containing approximately 18-20 nucleotides (encoding about a 6-7 amino acid stretch) are prepared and used to probe genomic DNA libraries to obtain hybridization under conditions of sufficient stringency to eliminate false positives using procedures well known in the art. (See Sambrook *et al*. Molecular Cloning, Cold Spring Harbor Press 1989)

DNA segments (i.e., synthetic oligonucleotides) that are used as probes or specific primers for the polymerase chain reaction (PCR), as well as gene sequences encoding methioninase, can readily be synthesized by chemical techniques, for example, the phosphotriester method of Matteucci, *et al*., (J. Am. Chem. Soc. 103:3185-3191, 1981) or using automated synthesis methods. In addition, larger DNA segments can readily be prepared by well known methods, such as synthesis of a group of oligonucleotides that define the DNA segment, followed by hybridization and ligation of oligonucleotides to build the complete segment.

In addition to PCR and DNA probe based methods, DNA molecules encoding methioninase can be isolated using polyclonal antiserum or monoclonal antibodies raised against peptide fragments of Figure 8 that are predicted as being immunogenic. Such antibodies can be used to probe an expression library generated from a given organism, such as a lambda gtll library, to obtain DNA molecules encoding methioninase from an organism other the *P. putida*.

Once a naturally occurring methioninase-encoding nucleic acid molecule is obtain, a skilled artisan can readily employ random or site specific mutagenesis procedures to alter the methioninase-encoding sequence so as to increase the level of expression or to substitute, add, or delete one or more amino acids from the encoded methioninase.

In one embodiment, the methioninase-encoding sequence is altered so as to increase the level of expression of the reconbinant methioninase in a given host cell without changing the amino acid sequence of the encoded methioninase. Increased expression of recombinant methioninase in a particular host can be obtained by altering one or more of the codons present in the nucleic acid molecule so that the resulting codons are ones that are more frequently used by the host organism to encode a particular amino acid. Altering a nucleotide sequence to contain preferred codons can be accomplished using art known procedures such as site directed mutagenesis or by synthesizing a nucleic acid molecule containing the preferred codons.

In addition to alterations that affect expression, methioninase-encoding nucleic acid molecules can be altered so as to facilitate purification of the resulting protein. For example, as disclosed in the Examples, by altering either the amino or carboxy terminus of the recombinant methioninase so as to add a polyhistidine stretch, Ni⁺⁺ sepharose can be used to purify the resulting fusion protein.

The methioninase-encoding sequence can also be altered to introduce changes in the amino acid sequence of the encoded methioninase, so as to add, substitute, or delete one or more amino acid residues. The resulting recombinant methioninase will preferably contain alterations that result in recombinant methioninase with better biological or physiological properties such as increased activity, decreased Kₘ, decreased immunogenicity, or increased serum half life. Such altered forms can be rationally designed or randomly generated.

An alteration is said to be rationally designed when the alteration is specifically chosen based on the amino acid sequence of the starting and resulting proteins and a desired physiological property. For example, one type of rationally designed alteration is to replace hydrophobic amino acids with less hydrophobic residues to increase solubility. The preferred method for generating rationally designed alterations is site direct mutagenesis using a mismatched PCR primer extension method.

Alterations are said to be randomly generated when the alteration is not rationally selected. Random mutagenesis techniques, such as chemical mutagenesis, PCR shuffling and linker scanning mutagenesis, generate a large variety of random and non-specific alterations in a given protein encoding sequence. Such methods can be used to radically alter the methioninase-encoding nucleic acid molecule.

Altered forms of recombinant methioninase generated in this fashion are then screened for desired properties using a variety of art known methods. The choice of selection method employed will be dependent of the host, vector, and mutagenesis methods employed as well as the properties that are selected for.

The present invention further provides vectors containing one or more of the expression modules of the present invention. Vectors are DNA molecules that are capable of autonomous replication within a host. Vectors can contain an episomal origin of replication derived from a naturally occurring plasmid, a genomic origin of replication, or can be derived from a viral genome. The choice of the vector to which an expression module of the present invention is inserted depends directly, as is well known in the art, on the functional properties desired, e.g., protein expression, and the host cell to be transformed.

In one embodiment, the vector includes a prokaryotic replicon. Prokaryotic replicons such as the ColEl replicon, are well known in the art and can readily be employed in combination with an expression module of the present invention. In addition, the vector may include a gene encoding a selectable marker such as a drug resistance.

Eukaryotic expression vectors can also be used in combination with an expression module of the present invention. Eukaryotic cell expression vectors are well known in the art and are available from several commercial sources. Typical of such vectors are PSVL and pKSV-10 (Pharmacia), pBPV-1/pML2d (International Biotechnologies, Inc.), pTDT1 (ATCC, #31255), the vector pCDM8 described herein, and the like eukaryotic expression vectors. High level expression vectors can further be generated using insect cell expression systems such as a bacculovirus based vector system.

In general terms, the generation of a high expression module encoding methioninase typically involves the following:

First, a DNA is obtained that encodes methioninase. If the sequence is uninterrupted by introns, as expected from a bacterial source, it is suitable for expression in any host. This sequence may be altered to be in a readily excisable and recoverable form by inserting sequences containing one or more restriction endonuclease sites at regions flanking the methioninase-encoding sequence.

The excised or recovered coding sequence is then placed in operable linkage with a high expression control element, preferably in a replicable expression vector. The expression module or vector is then used to transform a suitable host and the transformed host is cultured under conditions to effect the production of the recombinant methioninase. Optionally the recombinant methioninase is isolated from the medium or from the cells; recovery and purification of the protein may not be necessary in some instances, where some impurities may be tolerated.

Each of the foregoing steps can be done in a variety of ways. For example, the desired coding sequences may be obtained from genomic fragments and used directly in appropriate hosts. The constructions of expression vectors that are operable in a variety of hosts are made using two or more appropriate replicons and control elements. Suitable restriction sites can, if not normally available, be added to the ends of the coding sequence so as to provide an excisable gene to insert into these vectors.

### II. Transformed Host Cells Expressing High Levels of Recombinant methioninase

The present invention further provides host cells transformed with an expression module or vector of the present invention so as to produce from about 5-75% of total cellular protein as recombinant methioninase, preferably more than about 10% of total cellular protein. The host cell can be either a prokaryotic or a eukaryotic host.

Any prokaryotic host can be used to express the high-level methioninase-encoding modules of the present invention. The preferred prokaryotic host is *E. coli.* In the Examples that follow, the DH5α and BL21(DE3) strains of *E. coli* were used.

Preferred eukaryotic host cells include insect cells, yeast cells and mammalian cells, preferably insect cells such as SP6 and vertebrate cells such as those from a mouse, rat, monkey or human fibroblastic cell line. Other preferred eukaryotic host cells include Chinese hamster ovary (CHO) cells available from the ATCC as CCL61, NIH Swiss mouse embryo cells NIH/3T3 available from the ATCC as CRL 1658, baby hamster kidney cells (BHK), and the like eukaryotic tissue culture cell lines.

Transformation of an appropriate host with a high-level expression recombinant module of the present invention is accomplished by well known methods that typically depend on the type of host and vector used. With regard to transformation of prokaryotic host cells, electroporation or salt treatment of the host cells is preferred, for example, see Cohen *et al*., Proc. Natl. Acad. Sci. USA 69:2110, 1972; and Maniatis *et al*., Molecular Cloning, A Laboratory Mammal, Cold Spring Harbor Laboratory, Cold Spring Harbor, NY (1982).

With regard to transformation of eukaryotic cells, electroporation or the use of a cationic lipid is preferred, for example, see Graham *et al*., Virol. 52:456, 1973; and Wigler *et al*., Proc. Natl. Acad. Sci. USA 76:1373-76, 1979.

Successfully transformed cells, i.e., cells that contain an expression module of the present invention, can be identified by well known techniques. For example, cells resulting from the introduction of an expression module of the present invention can be cloned to produce single colonies. Cells from those colonies can be harvested, lysed and their DNA content examined for the presence of the rDNA using a method such as that described by Southern, J. Mol. Biol., 98:503, 1975, or Berent *et al*., Biotech. 3:208, 1985. However, as described below, the present invention further provides a rapid screening method to identify transformants which express high levels of recombinant methioninase.

### III. Identification of Hosts Expressing High Levels of Recombinant methioninase

The present invention further provides methods of identifying a transformed host cell which produces recombinant methioninase at levels from about 5-75% of total cellular protein. Specifically, it has been observed that transformed host cells expressing from about 5-75% of total cellular protein as recombinant methioninase, have a distinct and observable pink color. This is particularly pronounced when *E. coli* is used as the host.

To identify a transformed host cell expressing high levels of recombinant methioninase, a transformed cell is grown on or in a media under conditions in which the recombinant methioninase is expressed and that allows visual inspection of the growing cells. The growing cells or colonies are examined and selected based on the displaying of a pink color.

A variety of culture/growth conditions can be employed to grow transformed host cells for selection using the present methods. The components of the growth medium will depend on the nutritional requirements of the host/vector system employed and inspection system used to identify the pink color associated with high levels of recombinant methioninase expression and thereby allowing isolation of a high-level expression clone. The preferred medium is a solid medium onto which the transformed host cells can be plated and grown as isolated colonies, each of which is derived from single host. The preferred method of identifying high-level expression clones is visual inspection of growing colonies.

### IV. Production of Recombinant Methioninase Using a High-Level Expression Module

The present invention further provides methods for producing recombinant methioninase. Specifically, recombinant methioninase can be produced at commercially significant levels using a host transformed with one or more of the high-level expression modules of the present invention. Such a transformed host will express recombinant methioninase at a level from about 5-75% of total cellular protein. Using the hosts of the-present invention, a skilled artisan can readily produce recombinant methioninase for use in a variety of diagnostic and therapeutic methods using art known methods.

The preferred method for purifying recombinant methioninase produced using a transformed host containing a high expression module encoding methioninase, or from a host that naturally produces methioninase, comprises the steps of:
a) heating an extract of a transformed cell that contains methioninase in aqueous buffers from about 40-60°C for about 1-10 min., preferably 50°C for 1 min.;
b) centrifugation of the heated extract from about 10k to 20k rpm in a GS-3 rotor (Sorvall, Du Pont) for about 15 min. to 1 hour, preferably at about 13K rpm for about 30 min. at 4°C;
c) ultrafiltration of the supernatant using a filter of about 50K to 100K pore size, preferably a Millipore Pre/Scale:TFF PLHK 100 K 2.5 ft² cartridge using a 10mM potassium phosphate buffer (pH8.3);
d) DEAL ion exchange chromatography in low ionic strength (from about 10-50mM) KCl in a 10-20 mM potassium phosphate buffer at about pH 7.0-7.6, and collecting fractions containing methioninase eluted in a 40-200mM KCl gradient, preferably using DEAE-Sepharose FF column;
e) a second DEAE ion exchange chromatography in medium ionic strength (50-100mM) KCl in a 10-20 mM potassium phosphate buffer at about pH 8.0-8.6, and collecting fractions containing methioninase eluted in a phosphate buffer (pH 8.3) eluted in 100-230 mM KCl, preferably using DEAE-Sepharose FF column; and
f) contacting said fractions collected in step (e) with a chromatography medium capable of absorbing endotoxin, and collecting the eluant, thereby removing endotoxin from said eluant to form endotoxin-free methioninase having at least 20 units methioninase activity per milligram protein and from 1-100 ng of endotoxin per mg protein, preferably using an Acticlean® Etox column.

The cell extract is preferably prepared from a host cell that has been altered to express high levels of recombinant methioninase (from about 5-75% of total cellular protein). For bacterial cell extracts, the extracts are generally prepared by first harvesting and washing bacterial cell cultures to form a cell paste/pellet, depending upon whether harvesting is by centrifugation or by hollow fiber filtration, which methods are generally well known.

The cells are then disrupted using conventional means. Preferably the cells are disrupted using a homogenizer, such as a cavitator-type homogenizer, for example, a Microfluidics Corp. Model #HC8000.

The resulting suspension is heated to precipitate selective proteins and other insoluble materials. Typical heating conditions are from about 45-60°C for 1-10 minutes. Preferred is a heating step of 50°C for 1 minute.

The heated extract is centrifuged to remove debris, and the supernatant is filtered and applied to DEAE ion-exchange chromatography medium in two steps as described above. Preferred adsorption and elution conditions are described in the Examples. Any of a variety of DEAE ion exchange column chromatography media can be used in these steps, and the choice of media is not to be construed as limiting. Commercial sources include Pharmacia Fine Chemicals, BioRad, and Sigma.

Thereafter, endotoxin is removed to produce a protein having acceptable levels of endotoxin as recited earlier. The endotoxin removal step can be carried out in any of a variety of means, as are well known, and typically involve contacting the protein in solution with a chromatography medium capable of adsorbing endotoxin, and yielding a chromatography medium eluant which contains endotoxin-free protein. The preferred commercial reagent for use in removing endotoxin is Acticlean® Etox.

### G. Use of Methioninase for Prevention of Hyperhomocysteinemia-associated Cardiovascular Disease

Another aspect of the invention is the use of methioninase for homocysteine depletion therapy.

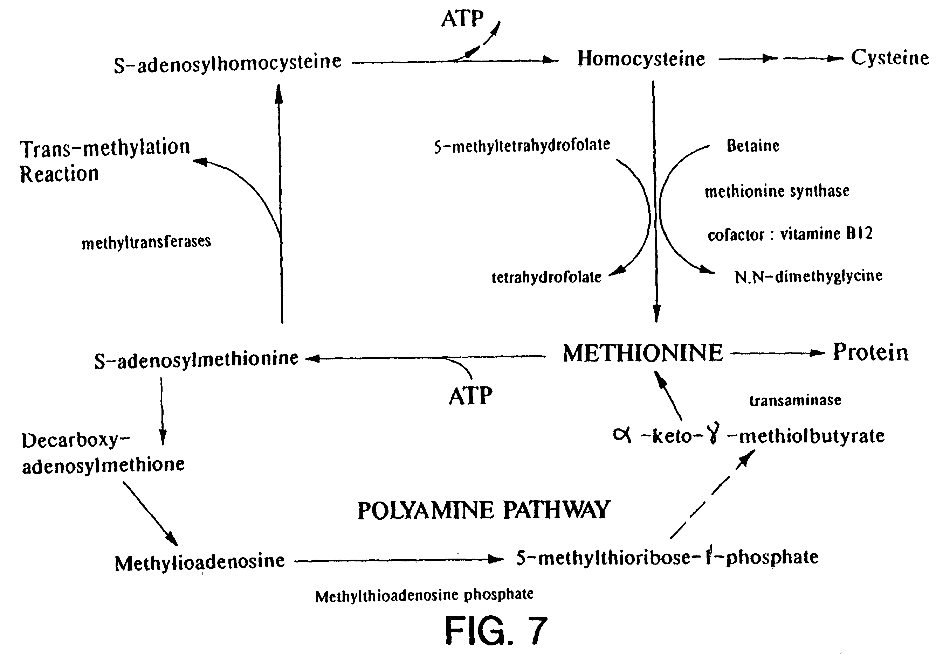

Hyperhomocysteinemia has been associated with various types of cardiovascular diseases. The first association of these diseases with homocysteine was made by McCully in 1969. (McCully, KS, Am. J. Pathol. 56: 111-28, 1969) McCully found a link between elevated plasma homocysteine concentrations and arteriosclerotic diseases. A recently concluded study of 1,041 people from the Framingham Heart Study found that elevated plasma levels of homocysteine leads to an increased risk of arteriosclerosis (Selhub, J. et al., N. Engl. J. Med. 32:286-91, 1995). Other studies have linked even moderate hyperhomocysteinemia to peripheral vascular, cerebrovascular and coronary heart disease. (Kang, S. et al., Annu. Rev. Nutr. 12:279-98, 1992). For example, fasting homocysteine concentrations in patients with vascular disease are 31% higher than in normal subjects. Plasma homocysteine concentrations 12% above the upper limit of normal were associated with the 3.4-fold increase in risk for myocardial infarction. (Stampfer, M. et al., JAMA 268:877-81, 1992). Studies of homocysteine metabolism have found that in 20 case control and cross-sectional studies of over 2000 subjects, show that patients with stroke and other cardiovascular blood diseases have higher blood levels of homocysteine than subjects with no cardiovascular diseases. This is in contrast with the fact that most patients with myocardial infarction have normal cholesterol levels. A striking result was found in the Physicians Health Study, where it was shown prospectively that where blood was drawn before cardiovascular disease was diagnosed 271 men who later had myocardial infarctions had significantly higher mean base line levels of homocysteine than matched controls who did not have myocardial infarctions (Ueland, P. et al., Cardiovascular Disease Hemostasis and Endothelial Function, New York: Marcel Dekler; 183-236, 1992). Although a variety of conditions can lead to elevated homocysteine levels, the relation between high levels of homocysteine and vascular disease is present regardless of the underlying metabolic cause. In a direct study, vascular lesions were induced in baboons and the baboons were infused with homocysteine for three months (Ueland, P., et al, *supra*). Hyperhomocysteinemia may be diagnosed by oral administration of methionine followed by the measurement of the levels of homocysteine in the patients. Abnormal homocysteine plasma concentrations after this oral methionine challenge is twelve times more present in patients with arteriosclerotic disease than in normal subjects (Ueland, et al., *supra*). Plasma homocysteine levels can be easily and rapidly measured by HPLC assays. Studies suggest that 40% of the population may have elevated homocysteine levels and be at risk for cardiovascular disease (Stampfer, M. and Malinow, M., New Engl. J. Med. 332:326-329 1995). Hyperhomocysteinemia can have acute effects in inducing arteriosclerotic disease and putting individuals at risk for myocardial infarction and cerebrovascular disease. Acute medical intervention is thus indicated for a large fraction of individuals at risk for cardiovascular disease. The methioninase of the present invention may be used as the therapy of choice for acute intervention to immediately lower homocysteine levels in individuals at risk. Methioninase catalyzes two reactions, the cleavage of both the nitrogen-carbon bond and gamma-carbon-sulfar bond not only in methionine but also in homocysteine (Hoffman, Bioch. et Biophys. Acta, Reviews on Cancer, 738:49-87, 1984). Figure 7 illustrates the metabolic cycle of homocysteine and methionine metabolism. The vitamins B-12, B-6, and folate influence the "left" side of the cycle illustrated in the figure and will be helpful in the maintenance of normal homocysteine levels in some individuals after methicninase treatments. However, the "right" side of the cycle is independent of the levels of these vitamins. In the right side of the cycle the presence of methionine can lead to excess hcmocysteine levels via elevated transmethylation reactions which may lead to cancer as well as to arteriosclerotic disease. For individuals with abnormalities in the right side of the cycle, maintenance as well as acute use of methioninase may be necessary to maintain normal levels of homocysteine. For cardiovascular disease, the substrate specificity of methioninase for both methionine and homocysteine is critical: methioninase will lower both the methionine level which is a precursor of homocysteine and will also lower the homocysteine level directly. Previous work in the area has only suggested the use of vitamins B-12, B-6 and folate as therapy to lower hyperhomocysteinemia. As shown in Figure 7, merely providing these vitamins will not rectify any abnormalities in the right side of the cycle and may not decrease homocysteine in individuals with abnormalities in the right side of the cycle. The present invention includes a method of treating patients having such cardiovascular diseases with methioninase.

In one aspect, a method is provided of treating a patient having a cardiovascular disease comprising administering to the patient a therapeutically effective amount of methioninase.

A therapeutically effective amount of methioninase for homocysteine depletion is a predetermined amount calculated to achieve the desired level of homocysteine depletion so as to, for example, reduce the risk of arteriosclerosis.

Thus, the dosage ranges for the administration of methioninase of the invention are those large enough to produce the desired effect in which, for example, the risk of, or level of, arteriosclerosis, are reduced. The dosage should not be so large as to cause adverse side effects, such as hyperviscosity, syndromes, pulmonary edema, congestive heart failure, and the like. Generally, the dosage will vary with the age, condition, sex and extent of the disease in the patient and can be determined by one of skill in the art.

The dosage can be adjusted by the individual physician in the event of any complication.

A therapeutically effective amount of a methioninase of this invention is typically an amount such that when administered in a physiologically tolerable composition is sufficient to achieve a intravascular (plasma) or local concentration of from about 0.001 to about 100 U per ml, preferably above about 0.1 U, and more preferably above 1 U methioninase per ml. Typical dosages can be administered based on body weight, and are in the range of about 5-1000 U/kilogram (kg)/day, preferably about 10-50 U/kg/day, and more preferably about 20-40 U/kg/day.

In preferred methods, a methioninase used is substantially free of endotoxin, as discussed in detail above. Particularly preferred is the use of methioninase produced herein that is substantially free of endotoxin from a recombinant source.

Methods of administration known to those of ordinary skill in the art may be employed, and are described herein.

In another aspect of the invention a patient with a cardiovascular disease is treated by administration of a therapeutically effective amount of a methioninase which is substantially free of endotoxins and is conjugated to a polymer. In a preferred aspect, the polymer is polyethylene glycol. In another preferred aspect of the invention the cardiovascular disease is arteriosclerosis. In another aspect of the invention methioninase is administered to patients with extracranial carotid artery stenosis, peripheral vascular, cerebrovascular, coronary heart disease, and occlusive vascular disease.

In another aspect of the invention means are provided for treating a patient having hyperhomocysteinemia by administering to the patient a therapeutically effective amount of methioninase. The methioninase may be substantially free of endotoxins and the methioninase may be conjugated through a polymer such as polyethylene glycol.

The invention also provides means for lowering homocysteine levels in a patient comprising the step of administering to the patient a therapeutically effective amount of methioninase wherein the methioninase is substantially free of endotoxins. In another aspect of the invention a method is provided of preventing cardiovascular disease in a patient comprising the step of administering to the patient a therapeutically effective amount of methioninase. In a preferred aspect the methioninase administered to the patient is substantially free of endotoxin. In another preferred aspect the methioninase is conjugated to a polymer. In yet another preferred aspect a method is provided at preventing cardiovascular disease in a patient comprising the step of administering to the patient a therapeutically effective amount of a methioninase that is substantially free of endotoxin wherein the methioninase is also conjugated to polyethylene glycol. Example 7 provides one example of *in vivo* homocysteine depletion.

### H. Use of Methioninase for Tumor Imaging

In another aspect of the invention means provided of diagnosing a tumor in a patient comprising the steps of depleting ¹²C methionine in a patient by administering methioninase tc the patient followed by repleting the methionine in the patient by administering ¹¹C methionine to the patient and finally detecting the presence of ¹¹C methionine in the tumor cells of the patient. The ¹¹C methylation may be detected by means of, for example, but not limited to,. positron emission tomography (PET) scanning. Those of ordinary skill in the art are familiar with other methods of detecting ¹¹C uptake by cancerous cells. Such methods are described in, for example, Lapela et al., J. Nucl. Med. 35:1618-23, 1994; Miyazawa et al., J. Nucl., Med. 34:1886-91, 1993; Leskinen-Kallio et al., J. Nucl. Med. 33:691-95, 1992; Shields et al., J. Nucl. Med. 33:581-84, 1992; Huovinen et al., Brit J. Cancer 67:787-91, 1993; Dethy et al., J. Nucl. Med. 35:1162-66, 1994; Lindholm et al., J. Nucl . Med. 34:1711-16, 1993; Leskinen-Kallio et al., J. Nucl. Med. 32:1211-16, 1991: and Mineura et al., J. Nucl. Med.: 32726-28, 1991, all hereby incorporated by reference herein.

In a preferred aspect of the invention, the methioninase provided is substantially free of endotoxin.

In another preferred aspect of the invention the methioninase was conjugated to a polymer, such as polyethylene glycol.

### Examples

The following examples relating to this invention are illustrative and should not, of course, be construed as specifically limiting the invention. Moreover, such variations of the invention, now known or later developed, which would be within the purview of one skilled in the art are to be considered to fall within the scope of the present invention hereinafter claimed.

### Example 1

### Scaled Up Production of Methioninase

A strain of Pseudomonas putida was modified and selected for kanamycin resistance and for high levels of methioninase expression as follows:

ATCC 8209 Pseudomonas putida and ATCC 77100 E. coli were purchased from the ATCC in Oct. 1993. ATCC 77100 was grown in LB (Sambrook et al., Molecular Cloning: A laboratory manual A.l, 1989) with 50 ug/ml kanamycin. Plasmid pCN 51, a shuttle vector containing a kanamycin resistance gene that is able to replicate in both P. putida and E. coli (Nieco et al., Gene 87:145-149, 1990, hereby incorporated by reference herein was isolated from ATCC 77100 and purified using the Triton-lysozyme method (Sambrook et al., Molecular Cloning: A laboratory manual 1.29-1.30, 1989). ATCC 8209 was grown in LB medium and transformed with pCN 51 using standard transformation procedures such as those described in, for example, Sambrook et al., Molecular Cloning: a laboratory manual. 1.74-1.84, 1969. The Kanamycin-resistant strain was selected with kanamycin (100 ug/ml) in LB medium and further grown in LB plates (Sambrook et al., Molecular Cloning: A laboratory manual A.1-A.4, 1989) in 100 ug/ml kanamycin. A single colony was grown in 100 ug/ml kanamycin in LB overnight and then put under high-methioninase-expression conditions: 10% LB, 0.1% potassium phosphate buffer pH 7.2, 0.1% urea, 0.025% yeast extract, 0.01% magnesium sulfate and 0.25% methionine with 50 ug/ml kanamycin for 24 hours. The expression of methioninase was measured with the standard methioninase assay as described herein. The methioninase overproducing strain of Pseudomonas putida was selected and named as AC-1.

A scaled up fermentation process for AC-1 Pseudomonas putida was then employed. An AC-1 single colony was grown in an LB plate containing 50 µg/ml kanamycin. The selected colony was incubated in 5 ml LB containing 50 µg/ml kanamycin at 26°C with shaking at 250 rpm/min for 18 hours. Two ml of bacteria were amplified in 600 ml LB containing 50 µg/ml kanamycin at 26°C, with shaking at 200 rpm/min for 6 hours. Then 50 ml of bacteria were incubated in 2 liters LB containing 50 uM/ml kanamycin at 26°C with shaking at 100 rpm/min overnight (18 hours). Two liters of bacteria (OD₆₀₀1.2-1.6) were then grown in a 40-liter tank in a special medium which contained 10% LB, 0.1% potassium phosphate buffer pH 7.2, 0.1% glycerol, 0.1% urea, 0.025% yeast extract, 0.01% magnesium sulfate and 0.25% methionine under high aeration conditions at 26°C for 24 hours. The OD₆₀₀ reached 1.2-1.8 and the activity reached 20-30 units/liter. The optimal cell density for harvesting is an OD₆₀₀ of 1.5-1.8, with 2-3 mg/l of methioninase. This is equivalent to 1 kg of wet cells/400 liters. Preferably, a fully-equipped fermenter would be used with an approximate yield of 1 kg wet cells/10-20 liters. The cells were harvested with an AGT column (Model UFP-500-E-55 cartridge, A/G Technology Corporation) keeping the temperature at 4°C then centrifuged using an automatic refrigerated centrifuge (Sorvall superspeed RC2-B) at 4°C, at 9 krpm for 10 minutes. The cell pellet was then collected.

The cells were then suspended in extraction solution (20 mM potassium phosphate, pH 9.0) at a density of 500g wet cells/liter, and disrupted using three passages with a cavitator homogenizer (Microfluidics Corp. Model #HC 8000). The homogenate was stored at -800C immediately after cell breakage. The specific activity of methioninase in the homogenate was 0.08-0.1 units/mg protein.

The AC-1 homogenate was suspended in extraction buffer (10 mM potassium phosphate pH 7.2, 10 uM pyridoxal phosphate, 0.01% beta-mercaptoethanol, 1 mM EDTA and 20% ethanol and was heated at 50°C for 2 minutes. The heating step may be performed for any length of time sufficient to precipitate heat-sensitive contaminating proteins while maintaining methioninase activity. The suspension was centrifuged at 12 krpm at 4°C for 30 minutes. The supernatant was collected and ultrafiltered with a Millipore Prep/Scale- TFF PLHK 100k2.5 ft² cartridge. The pH was then adjusted to 7.2.

A sample of about 25-35 g of total protein in 10 L extraction buffer (10 mM potassium phosphate buffer pH 7.2 10 µM pyridoxal phosphate and 0.01% J-mercaptoethanol) was applied to a Toyopearl DEAE-650M column 10cm x 50cm (Toso Haas Japan) which was pre-equilibrated with 10 mM potassium phosphate buffer (pH 7.2). The column was pre-washed with 20-30 L of 40 mM potassium chloride in 10 mM potassium phosphate buffer, pH 7.2 containing 10 uM pyridoxal phosphate and 0.01% beta-mercaptoethanol, until the OD₂₆₀ dropped below 0.1. The column was then eluted with a linear gradient of potassium chloride at concentrations starting at 40 mM, increasing up to 300 mM in extraction buffer. Elution fractions of 400 ml were collected. The fractions containing methioninase were determined by methioninase activity assay as described herein and were pooled. The pooled fractions were pre-equilibrated with 10 mM potassium phosphate buffer, pH 8.3, containing the same concentration of pyridoxal phosphate and beta-mercaptoethanol, and 150 mM potassium chloride.

A sample of about 1-2 g of total protein obtained from the methioninase peak of the DEAE-650M (5cm X 20cm) column in buffer containing 10 mM potassium phosphate pH-8.3, 150 mM potassium chloride, 10 µM pyridoxal phosphate and 0.01% J-mercaptoethanol was applied to a DEAE-Sephadex A50-column. The column was eluted with a 150-500 mM KCl linear gradient in 10 mM potassium phosphate buffer, pH 8.3 containing 10 µM pyridoxal phosphate and 0.01% beta-mercaptoethanol. Elution fractions of 150 ml were collected. The fractions containing methioninase determined by activity assay as described herein were pooled. The pooled methioninase was then further purified to remove endotoxin. The sample was pre-equilibrated by dialysis in 0.12M sodium chloride and 10 mM sodium phosphate buffer, pH 7.1. The sample was applied to a 5cm X 15 cm Acticlean Etox resin column (Sterogen Bioseparations, Arcadia, CA). The enzyme was then eluted from the column using the same buffer, eluant fractions containing methioninase activity were collected, and the amount of endotoxin was then determined on the eluted fractions. Table 1 shows the results of this purification method.

**Table 1**

| Scale Up Of Methioninase Manufacturing Process | | | | | |
|---|---|---|---|---|---|
| Step | Volume (ml) | Activity Units/ml Total | Protein mg/ml (g) Total | Specific Activity (units/mg) | Yield PH (%) |
| Homogenate | 3850 | 1.05/5200 | 11.5/44.3 | 0.1/6.7 | 100 |
| Heat | 3130 | 1.43/4472 | 4.4/13.8 | 0.33/6.9 | 86 |
| Ultrafiltration | 2950 | 1.41/4160 | 4.25/12.5 | 0.33/7.2 | 80 |
| First Column | 850 | 4.42/3744 | 1.64/1.39 | 2.6/7.2 | 72 |
| Second Column | 1000 | 3.22/3220 | 0.22/0.22 | 14.6/8.3 | 62 |
| Third Column | 540 | 4.8/2620 | 0.34/0.18 | 14.3/7.1 | 52 |
| Concentration | 16.5 | 153/2529 | 10.5/0.173 | 14.8/7.1 | 48 |

An analysis of the purified methioninase on a 7.5% SDS-PAGE gel showed a single band of protein of 43 kd, representing one subunit of the 172 kd protein was observed when the active fractions from the second column were analyzed by SDS-PAGE. The purity of the methioninase isolated by this method was 98.7% as seen using HPLC analysis, which demonstrated only one peak of protein.

The extraction methods described herein resulted in a preparation of substantially isolated methioninase that was substantially free of endotoxin. Those of ordinary skill in the art will recognize that modifications may be made to the extraction methods described in and are included within the scope of the invention.

### Example 2

### Dependence of Human Tumors on Elevated Levels of Methionine

Methionine dependence occurs frequently in human cancer. Twenty NCI human tumor cell lines from all major organ systems were tested for methionine dependence. All twenty cell lines were found to be methionine dependent. In addition, fresh human tumor specimens were tested and found to be methionine dependent. Normal human cell lines were not methionine dependent.

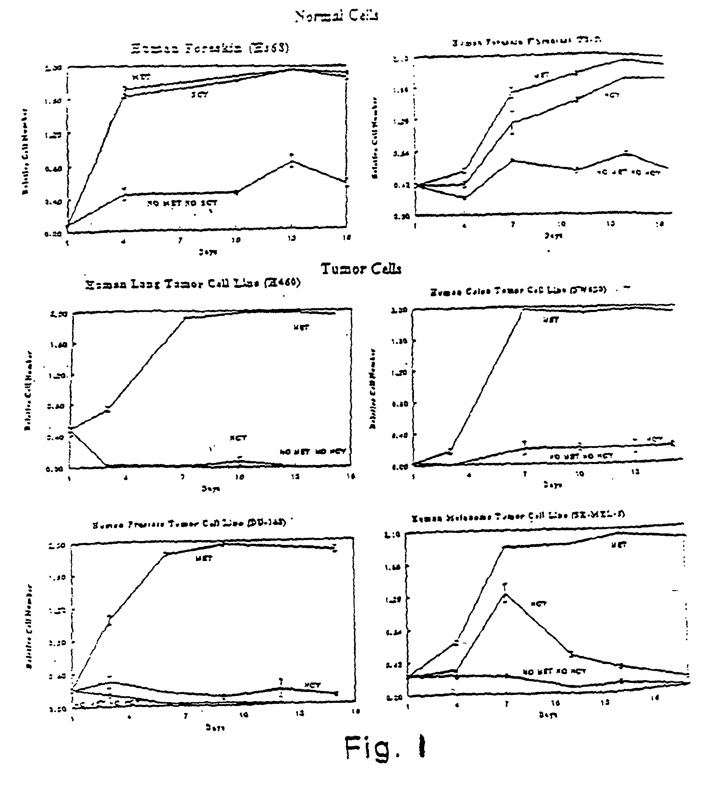

Figure 1 shows the results of one such test. In Figure 1, normal cell strains and tumor cell lines were grown in Eagle's MEM with 10% dialyzed serum that was either: methionine-containing homocysteine-free (MET⁻HCY⁻); methionine-free, homocysteine-containing (MET⁻HCY⁻) or methionine-free, homocysteine-free (MET⁻HCY⁻). Kidney, colon, lung and prostate cancer cell lines and melanoma and normal fibroblast strains were screened in methionine (MET)-containing medium, MET-free medium, homocysteine (HCY)-containing medium and in MET-free medium, homocysteine (HCY)-containing medium and in MET-free, HCY -free medium. The cells were grown in Eagle's Minimum Essential medium (MEM medium) supplemented with 10% dialyzed fetal bovine serum, 10 µM hydroxocobalamin and 100 uM folic acid. The medium was supplemented with or without methionine in the following way: MET+HCY: The medium was supplemented with 100 µM L-methionine without homocysteine. METHCY+: The medium was supplemented with 200 µM D,L-homocysteine without methionine. METHCY: The medium was methionine-free and homocysteine-free. The fetal bovine serum was dialyzed against phosphate buffered saline (PBS) (serum:PBS of 1:12) 10 times.

Cell proliferation was determined by metabolic reduction of the dye XTT as measured by absorption at 450 nm. Cells were grown on 48-well dishes by inoculation of between 5 X 10³ and 2 X 10⁴ cells per well. XTT (2,3-bis(2-methoxy-4-Nitro-5-sulfophenyl)-5-[(phenylamino)carbonyl]-2H-tetrazolium hydroxide), which is metabolically reduced in viable cells to a water soluble formazan product was used to determine cell proliferation. The absorbance of the reduced product was measured at 450 nm. XTT was prepared at 1 mg/ml in pre-warmed (37°C) PBS, Phenazine methosulfate (PMS) was prepared at 5 mM in PBS. Fresh XTT solution was mixed with PMS stock solution at a ratio of 5 11 PMS per 1 ml XTT. Each well was filled with 1 ml medium. 0.25 mL of mixed XTT were added to each well. The absorbance of reduced XTT was read at 450 nm with a Hitachi U-2000 spectrophotometer after 3 hours of incubation. The OD of XTT was measured every 3 days after the cells were cultured for an initial 24 hours. Foreskin fibroblast strains FS-3 and HS-68 proliferated essentially equally in MET⁺HCY⁻ or MET⁻HCY⁻. However, a new observation is reported here in that both fibroblast strains could proliferate to some degree and then maintain themselves for at least 16 days in MET HCY medium.

Table 2 illustrates the results of a cell proliferation assay of 20 tumor cell lines. In the assay, 5 x 10³ - 2 x 10⁴ cells/well of cell suspension were cultured in 48-well plates in MEM with 10% fetal bovine serum for 24 hours. Cells were washed with Hanks basic salt solution (HBSS) twice and then were divided into 3 groups. Group I: Cells cultured in MET⁺HCY⁻ medium; Group II: Cells cultured in MET⁻HCY⁺ medium; Group III: Cells cultured in MET⁺HCY⁻ medium. Cells were cultured in a gassed incubator with 95% air/5%CO₂. The media were changed every 2-3 days. In Table 2, strong growth of the cell lines is indicated with a +++, some growth with a ++, slight growth with a - and no growth with a -. Approximately one-half of the tumor cell lines could not grow in the MET-free HCY-containing medium with the rest growing to varying degrees whereas the same medium allowed the normal cells to grow as well as in MET-containing medium. Although the normal cell strains could grow to some extent and then maintain themselves for two weeks or more in MET-free, HCY-free medium, all 20 tumor cell lines tested died in this medium suggesting that potentially all cancers can be selectively killed by MET deprivation. This example provides evidence that methionine dependence may be a universal and. selective target for cancer therapy, especially with methioninase as the therapeutic.

**Table 2**

| Cell Lines | Type of Cancer Cell | Proliferation | | |
|---|---|---|---|---|
| | | MET⁻HCY⁻ | MET⁻HCY⁻ | MET⁻HCY⁻ |
| Tumor | | | | |
| A498 | Renal | + + + | - | - |
| CAKI | Renal | + + + | + + | - |
| SN12C | Renal | + + + | + | - |
| RXF-393 | Renal | + + | + | - |
| | | | | |
| DU- 145-1 | Prostate | + + + | - | - |
| PC-3 | Prostate | + + + | - | - |
| H322M | Lung | + + + | - | - |
| H23 | Lung | + + + | + + + | - |
| | | | | |
| SNB-75 | CNS | + + + | + + | - |
| SF-295 | CNS | + + + | + + | - |
| H460 | CNS | + + + | - | - |
| H522 | CNS | + + + | + + | - |
| | | | | |
| HCT-15 | colon | + + + | - | - |
| Colo 205 | Colon | + + + | + + + | - |
| SW-620 | Colon | + + + | - | - |
| HT29 | Colon | + + | + | - |
| | | | | |
| UACC-257 | Melanoma | + + + | + + + | - |
| UACC-62 | Melanoma | + + + | + + + | - |
| SK-MEL-5 | Melanoma | + + | + | - |
| Lox | Melanoma | + + | - | - |

| Normal Cells | | | | |
|---|---|---|---|---|
| FS-3 | Foreskin | + + + | + + + | + |
| HS-68 | Foreskin | + + + | + + + | + |

### Example 3

### Inhibition of Human Tumor Growth by Methioninase in Mouse Xenograft Models

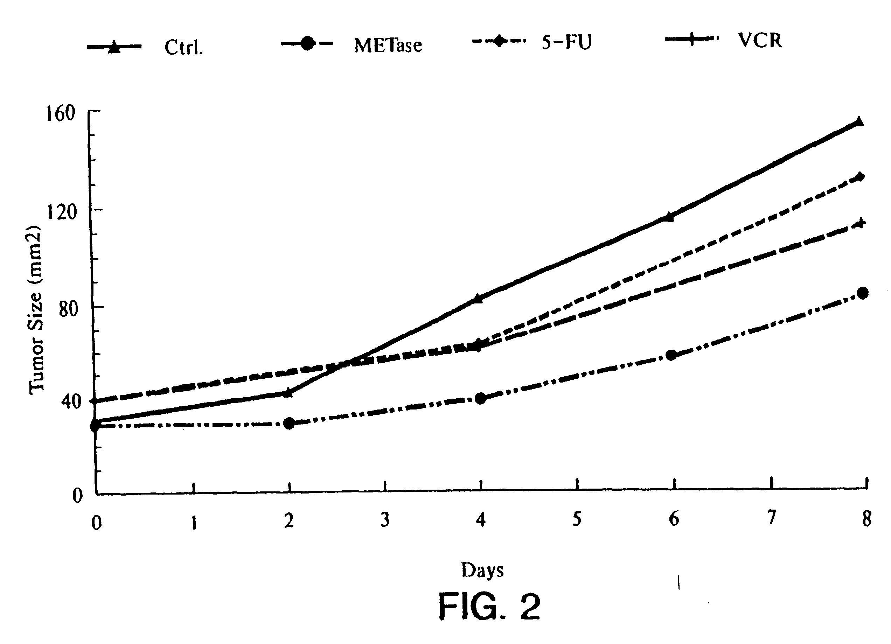

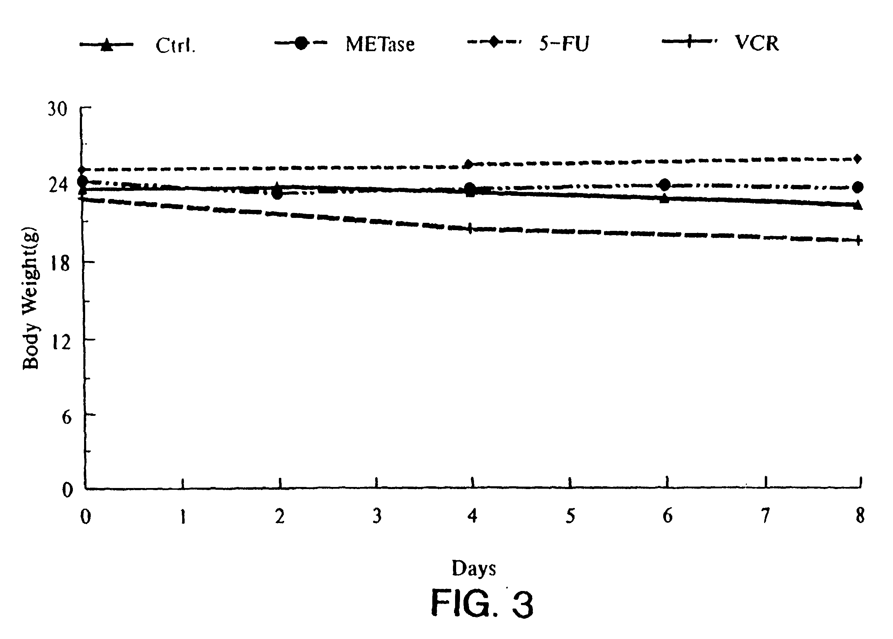

To test the efficacy of methioninase on the reduction of tumor growth, a mouse xenograft model was used. A human lung cancer tumor (H460) was transplanted in the subcutis of nude mice. The mice were divided into four groups of four mice each. Four days after the transplantation, group A was administered 0.4 ml buffer (0.12 M sodium chloride, 10 mM sodium phosphate pH 7.1) by intraperitoneal injection every 4 hours; group B was administered methioninase (1 unit/g) by intraperitoneal injection every 4 hours; group C was administered 5-FU (60mg/kg) by intraperitoneal injection every 4 days; and group D was administered with vincristine (VCR) (0.9mg/kg) by intraperitoneal injection every 4 days. Tumor size and body weight were measured every two days. The results are shown in Figures 2 and 3. The results indicate that treatment with methioninase greatly slowed the growth of the H460 human non-small-cell-lung cancer in nude mice. In these experiments, the mice were fed normal, not methionine-depleted, diets. The efficacy of methioninase against H460 was in stark contrast to 5-fluorouracil and vincristine treatment, which were inactive against this tumor. The activity of the administered methioninase did not cause weight loss for up to 10 days treatment at 40-120 units/day indicating the possibility of low toxicity. In contrast, vincristine caused weight loss, indicating toxicity. Thus, methioninase is a highly effective antitumor agent with a new tumor-selective mode of action with minimal toxicity, demonstrating potential clinical effectiveness against human solid tumors.

### Example 4

### Preparation of PEG-methioninase

Methoxy polyethylene glycol succinimidyl carbonate, (M-SC 5000 PEG), molecular weight 5000, was dissolved in 20 mM sodium phosphate buffer (pH 8.3) at a concentration between 2 mM and 20 mM. The molar ratios of M - SC 5000 PEG to methioninase were varied from 6:1 to 240:1. The PEGylation reactions were carried out in reaction buffer (25 mM sodium phosphate buffer, pH 8.3), at 4°C or 20°C from 30 minutes to 60 minutes. The reactions were stopped with stop buffer (0.14 M sodium phosphate buffer, pH 6.5) at 0°C. Unreacted M - SC 5000 PEG was then removed by gel filtration chromatography as described herein. The resulting PEG - methioninase was formulated in 0.12 M sodium chloride and 10 mM sodium phosphate (pH 7.2) with the use of 30k Amicon Centriprep concentrators. The PEG-methioninase was then sterilized by filtration with a 0.2 uM micron membrane filter. The PEG-methioninase may be stored at -70°C for up to 6 months without loss of activity.

Methioninase activity was measured. The activity of PEG-methioninase was maintained to at least 60% of the activity of nonPEGylated methioninase. PEG-methioninase was analyzed by both native and SDS polyacrylamide gel electrophoresis as described by Laemmli and by Sambrook (Laemmli, Nature 227-680, 1970; Sambrook et al., Molecular Cloning: a laboratory manual 18.47-18.59, 1989) and modified as follows: Electrophoresis of PEG-methioninase was carried out in 7.5% polyacrylamide-precasted gels in 0.2 M Tris-glycine buffer (pH 8.3), with or without SDS. The gels were stained with Coomassie blue and destained with 40% methanol 10% acetic acid. No unmodified methioninase band was seen when PEG-methioninase was applied to native gels, although a very small band could be seen in SDS gels.

PEG-methioninase was then analyzed by an HPLC gel filtration column as follows: PEG-methioninase (20 11 at 1-2 mg/ml) was applied using a 20 µm loop to a Progel-TSK G3000 SW (Supelco) column in 0.2M sodium phosphate buffer (pH 7.2) and eluted with 0.2M sodium phosphate buffer (pH 7.2) at a flow rate of 1.0 ml/mn. Only one peak of protein was detected, the PEG-methioninase peak, which was free of unreacted PEG; no nonPEGylated methioninase peak could be detected. The purity of PEG-methioninase was approximately 100%.

### a) Time Course Studies of the PEGylation Reaction

The time course of the conjugation of polyethylene glycol to methioninase was measured as follows: 0.07 mM of methioninase was reacted with 0.4 mM of M - SC 5000 PEG in 0.2 M sodium phosphate buffer ( pH 8.3 ) for various time periods up to 2 hours at 4°C. The reactions are stopped at various time points with 0.2 M sodium phosphate pH 6.5 ). Non - reacted PEG was removed with a 30k Amicon centriprep concentrator. The results are shown in Table 3.

PEG-methioninase was analyzed on native and SDS-PAGE gels as described herein. The appearance of a higher molecular weight band was measured and is indicated with -, +-, +, or ++ symbol in Table 3, wi th - indicating the lack of PEG-methioninase or methioninase, +- indicating small amounts of FEG-metrioninase or methioninase, and ++ indicating large amounts of FEG-methioninase or methioninase. Table 3 also indicates the activity of the PEG-methioninase or methioninase.

**Table 3**

| Time Course of Methioninase Pegylation | | | | | | | | | |
|---|---|---|---|---|---|---|---|---|---|
| | Molar Ratio | Condition | | Time Course (Min) | | | | | |
| | (M*:P**) | °C | pH | 0 | 4 | 30 | 60 | 90 | 120 |
| Activity(%) | 1:6 | 4 | 8.3 | 100 | 99 | 77 | 70 | 60 | 58 |
| Native PAGE | Methioninase | | | ++ | + | +- | - | - | - |
| | PEG-methioninase | | | - | + | + | ++ | ++ | ++ |
| SDS PAGE | Methioninase | | | ++ | ++ | ++ | + | + | +- |
| | PEG-methioninase | | | - | +- | + | + | ++ | ++ |

| | | | | | | | | | |
|---|---|---|---|---|---|---|---|---|---|
| *M=methioninase | | | | | | | | | |
| **P=M-SC 5000-PEG. | | | | | | | | | |

### b) Concentration dependence of Methioninase PEGylation with the M-SC 5000 PEG

0.07 mM of methioninase was reacted with various concentrations (0.4 mM to 4.0 mM) of M - SC 5000 PEG in 0.2 M sodium phosphate buffer (pH 8.3). The molar ratios were 1:6 - 1:60. The reactions were carried out at the same conditions as in Example a for 60 minutes at 4°C.

The results are shown in Table 4.

**Table 4**

| Concentration Dependence of Methioninase Pegylation | | | | | | | | |
|---|---|---|---|---|---|---|---|---|
| | Condition | | Molar Ratio (P**:M*) | | | | | |
| | °C | min | 0 | 6 | 12 | 24 | 48 | 60 |
| Activity (%) | 4 | 60 | 100 | 79 | 69 | 48 | 33 | 31 |
| Native-PAGE | M* | | ++ | - | - | - | - | - |
| | P-M*** | | | - | ++ | ++ | ++ | +++ |
| SDS-PAGE | M* | | ++ | + | + | +- | +- | +- |
| | P-M*** | | - | + | ++ | ++ | ++ | +++ |

| | | | | | | | | |
|---|---|---|---|---|---|---|---|---|
| *M: methioninase. | | | | | | | | |
| **P: PEG. | | | | | | | | |
| ***P-M: PEG-methioninase. | | | | | | | | |

### c) Pharmokinetics of PEG-Methioninase

Molar ratios of PEGylation of methioninase with M - SC 5000 PEG to methioninase were 1:6 ( P1 ) and 1 : 12 ( P2 ) respectively. The reactions were carried out at the same conditions as in (b) above. The purified P1 and P2 as well as unmodified methioninase were injected into the tail vein of three different groups of mice, respectively. The blood samples were collected at 0, 1, 2, 3, 4 and 20 hours. The activity assay of methioninase and PEG - methioninase and the level of depletion of methionine were measured. The results are shown in Table 5.

**Table 5**

| Pharmacokinetics of PEG-Methior inase | | | | | | | | |
|---|---|---|---|---|---|---|---|---|
| Mouse Group | | Treatment Agent Condition Dost Route | Methioninase Activity in Sera Half-life (Relative % hours) | | | | | |
| | | | 0 | 1 | 2 | 3 | 4 | |
| I | M' | Buffer 8 units G | 100 | 49 | 32 | 3 | 2 | - |
| II | F1" | M life 8 units | 100 | 66 | 36 | 4. | 1 | |
| III | P2" | Mip = life 8 units | 100 | 94 | 1 | 36 | 24 | |

| | | | | | | | | |
|---|---|---|---|---|---|---|---|---|
| 'M: methioninase. | | | | | | | | |
| "P, P1, P2: PEG-methioninase | | | | | | | | |

### Example 5

### Antigenicity experiment using PEG-methioninase in Guinea Pigs:

Methioninase was reacted with M - SC 5000 PEG at molar ratios of 1:60, 1:120 and 1:240, respectively, at 20°C for 30 minutes. The other conditions are the same as in (a) above. PEG-methioninase was analyzed by the methioninase activity assay, electrophoresis and HPLC. No band or peak of unmodified methioninase was detectable. Thus, PEG-methioninase was the only source of enzyme.

2 mg of methioninase or PEG-methioninase was administered intraperitoneally to guinea pigs every two days for three times total. Two weeks later, 4 mg of methioninase or PEG-methioninase was administered intravenously. 0.2 sodium phosphate buffer and BSA were used as negative and positive controls, respectively, in this experiment. The results are evaluated according to the criteria in Table 6. The results are shown in Table 7. PEGylation of methioninase results in a composition of extremely low antigenicity as tested in guinea pigs. Guinea pigs are known to be more sensitive than humans to antigens.

**TABLE 6**

| EVALUATION CRITERIA OF PEG-METHIONINASE ANTIGENICITY STUDY IN GUINEA PIGS | | | |
|---|---|---|---|
| 0. | Normal | 1. | Restlessness |
| 2. | Piloerection | 3. | Trembling |
| 4. | Running Nose | 5. | Sneezing |
| 6. | Coughing | 7. | Hyperpnea |
| 8. | Urination | 9. | Evacuation |
| 10. | Lacrimation | 11. | Dyspnea |
| 12. | Rhonchus | 13. | Cyanosis |
| 14. | Staggering gait | 15. | Jumping |
| 16. | Gasping & writhing | 17. | Convulsion |
| 18. | Side position | 19. | Cheyne-stokes respiration |
| 20. | Death | | |

| EVALUATION CRITERIA | | |
|---|---|---|
| (-) | negative | no change |
| (+) | slight | 1 - 4 |
| (++) | moderate | 1 - 10 |
| (+++) | severe | 1 - 19 |
| (++++) | death | 20 |

**Table 7**

| Antigenicity of PEG-ONCASE in Guinea Pigs | | | | | |
|---|---|---|---|---|---|
| Sensitization | | | Induction | | Evaluation |
| (Inject every two days, 3 times) | | (Two weeks later) | WHO Criteria | | |
| | Antigen | Doses | Antigen | Doses | |
| A | Buffer | 0.2 ml, ip | Buffer | 0.2 ml, ip | - |
| B | BSA | 1.2 ml, ip | BSA | 10 mg, iv | ++++ |
| C | Methioninase | 2.0 mg, ip | Methioninase | 4.0 mg, iv | ++++ |
| D | PEG-Methioninase | 2.0 mg, ip | PEG-Methioninase | 4.0 mg, iv | - |

The results showed that PEG-methioninase, in contrast to Bovine Serum Albumin (B.S.A.) and methioninase, has no antigenicity in guinea pigs, which suggests that PEG-methioninase is non-immunogeneic when administered to a mammal.

### Example 6

### Administration of Methioninase for Human Cancer Therapy

A Phase I dose-escalating study of methioninase has been started to determine methioninase toxicity, pharmacokinetics and maximum tolerated dose. A 2-hour infusion of 5,000 units (0.5g) and 10,000 units (1.0g) of methioninase has been administered by iv infusion for 2 hours into 2 patients with advanced breast cancer. Blood and urine samples were obtained at frequent intervals from 0 to 24 hours. The toxicity evaluations were carried out according to the WHO grading system. Pharmacokinetic data were obtained for both methioninase and methionine levels in the serum.

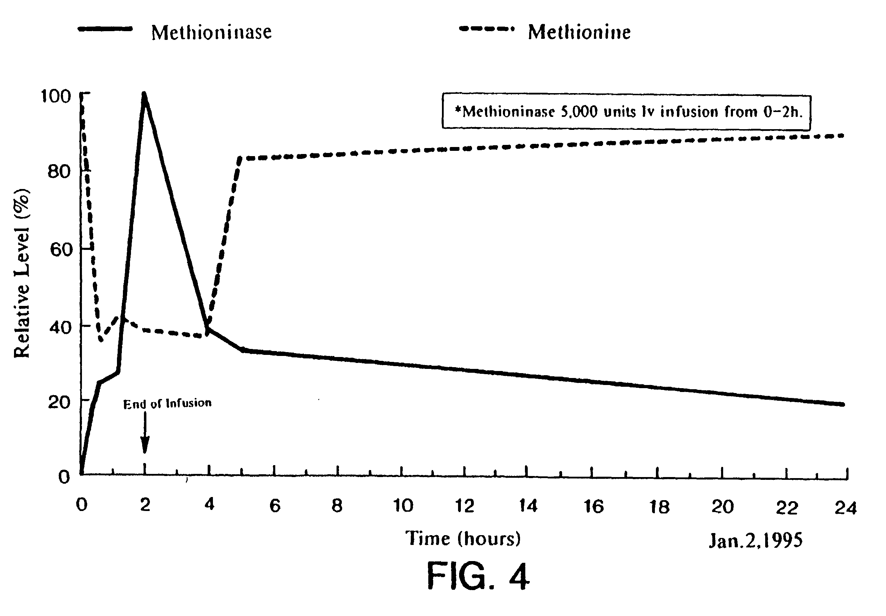

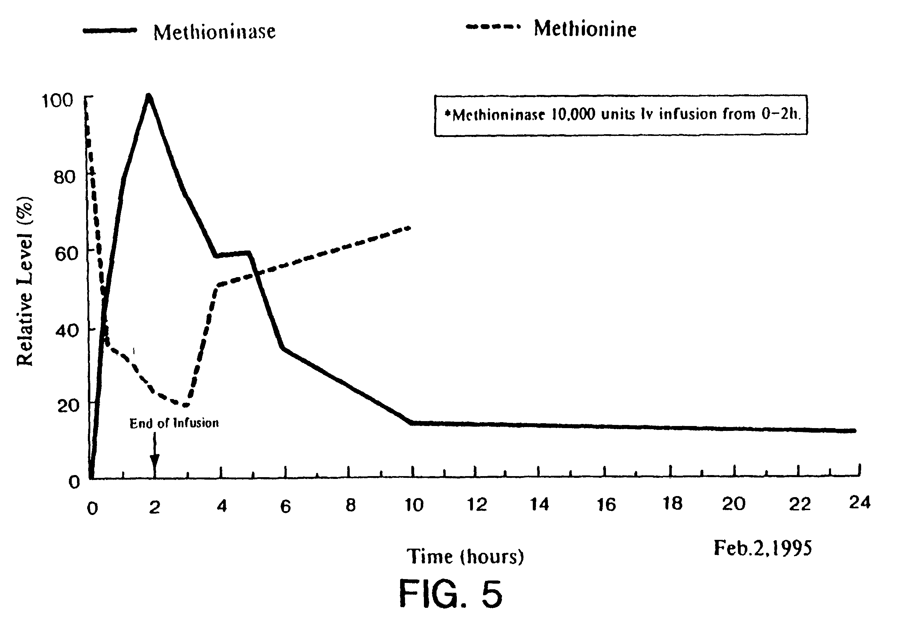

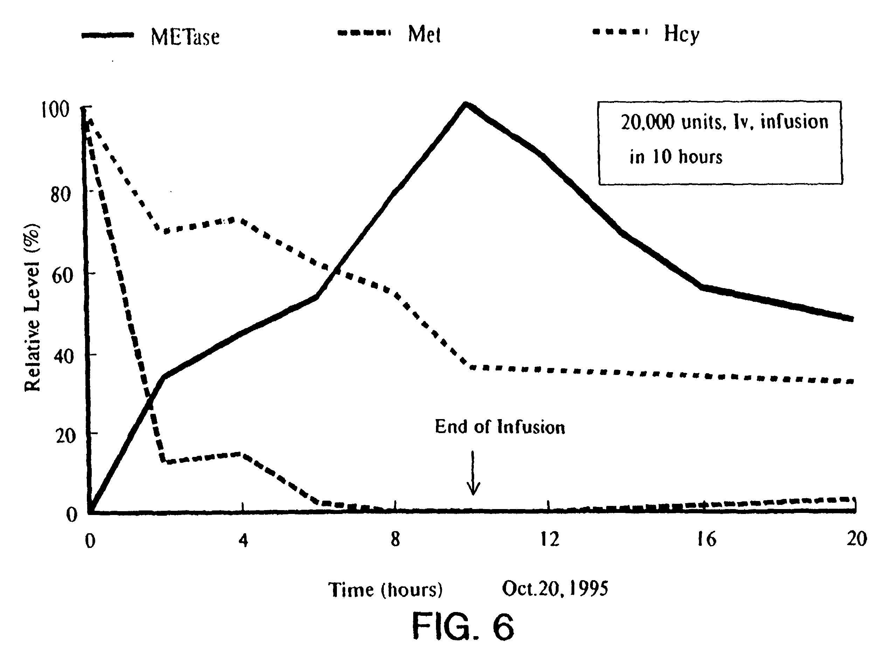

No acute clinical toxicity was observed whatsoever with all the toxicity criteria measured having a minimum grade of 0. The half-life of methioninase was 2 hours and 3.2 h, respectively, in patient 1 and patient 2. The depletion of serum methionine started within 30 minutes of the infusion, and was maintained for 4 hours after the infusion was completed. The lowest serum methionine levels were 35% and 19% of the pretreatment level, respectively, in patient 1 and patient 2. The results are shown in Tables 8-10 and Figures 4-6. The patients were treated as follows:
Patient 1: Date: December 14, 1994. Diagnosis: Breast cancer with axillary lymph node and lung metastasis. Female, 46 years old. 5,000 units (0.4 g) of methioninase purified according to extraction method (Example 1) was administered in a 200 ml intravenous infusion in 2 hours. Blood samples were collected before the treatment and after every two hours up to 20 hours. The levels of methionine and methioninase were measured and the relative levels calculated, Figure 4.
Patient 2: Date: February 2, 1995. Diagnosis: Breast cancer with axillary lymph node metastasis. Female, 54 years old. 10,000 units (0.8g) of methioninase purified according to Extraction Method (Example 1) was administered in a 400 ml intravenous infusion in 2 hours. Blood samples were collected before the treatment and after every two hours up to 20 hours. The levels of methionine and methioninase were measured and the relative levels calculated, Figure 5.
Patient 3: Date: October 15, 1995. Diagnosis: Breast cancer with axillary lymph node metastasis. Female. 45 years cld. 20,00 units (1.6 grams) of methioninase purified according to Extraction Method (Example 1) was administered in a 1000 ml intravenous infusion in 10 hours. Blood samples were collected before the treatment, and after every two hours up to 20 hours. The levels of methionine and methioninase were measured and the relative levels calculated, Figure 6.
Patient 3 maintained serum levels of methioninase as high as 50% of the maximum level for a subsequent 6 hours after infusion. Methionine depleted over 200-fold, from 23.1 uM to 0.1 uM, by the tenth hour of infusion. No clinical toxicity was observed whatsoever in all the toxicity criteria mearsued.

Toxicity evaluation was performed according to WHO toxicity criteria in four areas: a. History and diagnosis data; b. Physical examination; c. Laboratory evaluation; and d. Pharmacokinetics evaluation (methioninase and methionine levels in serum). The results, shown in Tables 8-11, and Figures 4-6, demonstrate that the methioninase of the present invention is not toxic and may be used to decrease methionine levels in humans.

**Table 8**

| Clinical Phase I Trial for Methioninase | | | |
|---|---|---|---|
| | Patient I | Patient II | Patient III |
| Diagnosis | Breast cancer with metastasis in axillary lymph node. Patient 1 had lung metastasis as well | | Breast cancer/metastasis |
| Sex | Female | Female | Female |
| Age | 46 | 54 | 45 |
| METase | 5,000 units | 10,000 units | 20.000 units |
| I.v. infusion | 2 hours | 2 hours | 10 hours |
| Blood collection | Before infusion and during infusion every two hours, after infusion 10 hours | | |
| Evaluation | WHO criteria | | |

**Table 9**

| Toxicity of Clinical Phase I Trial for Methioninase | | | |
|---|---|---|---|
| Physical & Laboratory | Toxicity Grade of FDA Standard | | |
| Examination | Patient I | Patient II | Patient II |
| Hematological | 0 | 0 | 0 |
| Gastrointestinal | 0 | 0 | 0 |
| Renal | 0 | 0 | 0 |
| Pulmonary | 0 | 0 | 0 |
| Fever | 0 | 0 | 0 |
| Allergic | 0 | 0 | 0 |
| Phlebitis | 0 | 0 | 0 |
| Cutaneous | 0 | 0 | 0 |
| Cardiac | 0 | 0 | 0 |
| Neurological | 0 | 0 | 0 |

**Table 10**

| Pilot Phase I Clinical Trial Methionine and Homocysteine Levels in Patient III (Methioninase 20,000 units iv infusion for 10 hours) | | | | | |
|---|---|---|---|---|---|
| Time | | Homocysteine | | Methionine | |
| | | µM | % | µM | % |
| Before infusion | | 7.7 | 100 | 23.1 | 100 |
| During infusion | 2h | 5.4 | 70 | 2.9 | 12.6 |
| | 4h | 5.6 | 73 | 3.4 | 14.7 |
| | 6h | 4.8 | 62 | 0.5 | 2.2 |
| | 8h | 4.2 | 55 | 0.2 | 0.09 |
| | 10h | 2.8 | 36 | 0.1 | 0.04 |
| After infusion | 2h | 2.6 | 34 | 0.2 | 0.01 |
| | 10h | 2.5 | 32 | 0.7 | 0.3 |

**Table 11**

| **VITAL PARAMETERS OF PATIENTS TREATED WITH METHIONINASE** | | | | | |
|---|---|---|---|---|---|
| **Patient** | **Treatment** | **Temperature** | **Pulse** | **Respiration Rate** | **Blood Pressure** |
| | Before | 36.7 | 134 | 21 | 110/70 |
| **# 1** | During (Max) | 36.8 | 138 | 22 | 120/80 |
| **O.R.L.** | (Min) | 36.4 | 125 | 24 | 110/70 |
| | After | 36.6 | 127 | 24 | 120/80 |
| | Before | 36.7 | 74 | 14 | 118/78 |
| **#2** | During (Max) | 36.6 | 74 | 12 | 120/80 |
| **S.N.K.** | (Min) | 36.6 | 70 | 14 | 110/70 |
| | After | 36.7 | 74 | 14 | 110/74 |
| | Before | 36.4 | 67 | 14 | 124/82 |
| **#3** | During (Max) | 37.0 | 76 | 14 | 128/74 |
| **S.N.O.** | (Min) | 36.0 | 66 | 12 | 90/70 |
| | After | 36.3 | 65 | 12 | 114/88 |

### Example 7

### In vi vo Depletion of Homocysteine

The *in vivo* effectiveness of methioninase for use in depleting homocysteine according to the present invention was demonstrated by administration of the enzyme to mice as follows: 4 units of methioninase purified according to the Method of Example 1 was injected intraperitoneally in mice. About one hour after the injection, the blood concentrations of methionine, homocysteine, and cysteine were assayed by subjecting 50 µl of mouse serum after derivitization with PITC, to analysis by reverse phase FPLC. The chromatographic profile was compared to that of PITC derivatized internal methionine, homocysteine, and cysteine standards. The sensitivity of the assay was about 0.5 µM methionine.

As shown in Table 12, compared to the levels of homocysteine and cysteine in control, untreated mice, methioninase had significant *in vivo* homocysteine depletion activity without any significant depletion of *in vivo* levels of cysteine.

**Table 12**

| *In Vivo* Reduction of Methionine and Effects on Other Amino Acids by Methioninase | | | |
|---|---|---|---|
| Sample | Methionine (µM) | Homocysteine (µM) | Cysteine (µM) |
| Control | 131.8 | 2.7 | 74 |
| Methioninase | 6.6 | 0.5 | 71 |

### Example 8

### Use of Methioninase for Tumor Imaging

[¹²C] methionine is depleted in a patient by the administration of purified methioninase as described herein. Preferably the methioninase is free of endotoxin. Methioninase (10,000-20,000 units) is administered by intravenous infusion over 4-8 hours. Approximately 5-50 mCi of [¹¹C] methioninase is then administered. Positron emission tomography (PET) imaging is then used to detect the uptake of the [¹¹C] tracer by the patient's cells. Tracer uptake is quantitated by calculating both the standardized uptake value (S.U.V.) and the kinetic influx constant (Ki) value by means known to those of ordinary skill in the art. Elevated levels of [¹¹C] methionine in cells indicates that such cells are likely to be tumor cells. The preferential [¹¹C] methionine uptake by the tumor cells provides a means of selectively detecting tumor cells among normal cells.

The foregoing specification, including the specific embodiments and examples, is intended to be illustrative of the present invention and is not to be taken as limiting. Numerous other variations and modifications can be effected without departing from the true spirit and scope of the present invention.

### Example 9

### Isolation of Nucleic Acid Molecules Encoding Methioninase PCR Reaction of the Insert of Methioninase Gene Clone:

Genomic DNA of *Pseudomonas putida* AC-1, derived from ATCC8209, was used as template; the primers used were as follows: 
t1:5'-GCCGGTCTGTGGAATAAGCT-3' (Sense), SEQ ID NO: 1
HindIII
 
t2:5'-CCAGGGTCGACTCCAGCGCC-3' (Antisense). SEQ ID NO: 2
Sal I
 The PCR reaction condition was as follows: first denaturation at 95°C for 10 minutes, then 5 cycles of denaturation at 94°C for 30 seconds, annealing at 60°C for 30 seconds, and extension at 72°C for 2 minutes; then 25 cycles of denaturation at 94°C for 30 seconds, 60°C for 30 seconds, then extension at 72°C for 1.5 minutes; then final extension at 72°C for 10 minutes. The PCR amplified products are two bands of which the 1365 bp band was collected, and purified as the insert ONCase-1 DNA.

### Cloning and Transformation

The ONCase-1 DNA was ligated with pT7Blue T-vector (Novagen) at the EcoR V T-cloning site. The pONCase-1 DNA was transformed into DHS-α bacterial cells using standard procedures.

### DNA Sequencing

DNA sequencing was performed using T7 DNA polymerase and the dideoxy nucleotide termination reaction. The primer walking method was used. [³⁵S] dATP was used for labeling. Sequencing reactions were analyzed on 6% polyacrylamide wedge or non-wedge gels containing 8M urea. DNA samples were loaded in the order of ACGT. DNA sequences were analyzed by MacVector. The DNA sequence and corresponding amino acid sequence are provided in Figure 8. (SEQ ID NO: 6)

### Example 10

### High Expression Clones of Recombinant Methioninase PCR Reaction of the Insert for the Methioninase Expression Clone:

The pONCase-1 clone was used as the template, the primers used are as follows: 
t14. 5' - GGAATTCCATATGCACGGCTCCAACAAGC - 3' (Sense)
NdeI
SEQ ID NO: 3
 
t15. 5' - AGTCATCCTAGGTCACATCATCATCATCATCATGGCACTCGCCTTGAGTGC-
3'
BamHI (Antisense) SEQ ID NO: 4
 
t18. 5' - AGTCATCCTAGGTCAGGCACTCGCCTTGAGTGC - 3' (Antisense)
BamHI
SEQ ID NO: 5
 The PCR reaction condition was as follows: first denaturation at 95°C for 10 minutes, then 5 cycles of denaturation at 94°C for 1 minute, annealing at 56°C for 1.5 minutes, and extension at 72°C for 2 minutes; then 20 cycles of denaturation at 94°C for 30 seconds, 56°C for 30 seconds, then extension at 72°C for 1.5 minutes; then final extension at 72°C for 10 minutes. Two PCR amplified products, ONCase-2 (1238bp), ONCase-3 (1220bp) band were collected and purified.

### Cloning and Transformation

The DNA of ONCase-2 and ONCase-3 DNA was digested with NdeI and BamHI and ligated with the pT7.7 vector at the NdeI and BamHI cloning sites. The pONCase-2 and pONCase-3 DNA sequences were then transformed into BL21 (DE3) bacterial cells using standard procedures.

### Selection of pAC-1 and pAC-2 Clones

The positive clones were selected from Ampicillin-containing plates. After storage at 4°C for 24 hours, the positive clones which expressed high level of recombinant methioninase had a distinct pink color that allowed their identification and selection. The methioninase expression levels of the positive clones were determined by activity assay. Two high expression clones were selected as the pAC-1 clone which contained ONCase-3 and as the pAC-2 clone which contained ONCase-2.

### Construction of pAC-3 Clone and pAC-4 Clone

The tetracycline resistance gene was obtained from pBR322 at the Ava I and Cla I sites. The Ava I end was filled into a blunt end, and was ligated with pAC-1 which was digested with the BamH I and Cla I restriction enzymes, with the BamH I end filled into a blunt end. Positive clones which became pink after storage at 4°C for 24 hours were selected from Tetracycline-containing plates. A high expression recombinant methioninase clone was determined by activity assay and named as the pAC-3 clone.

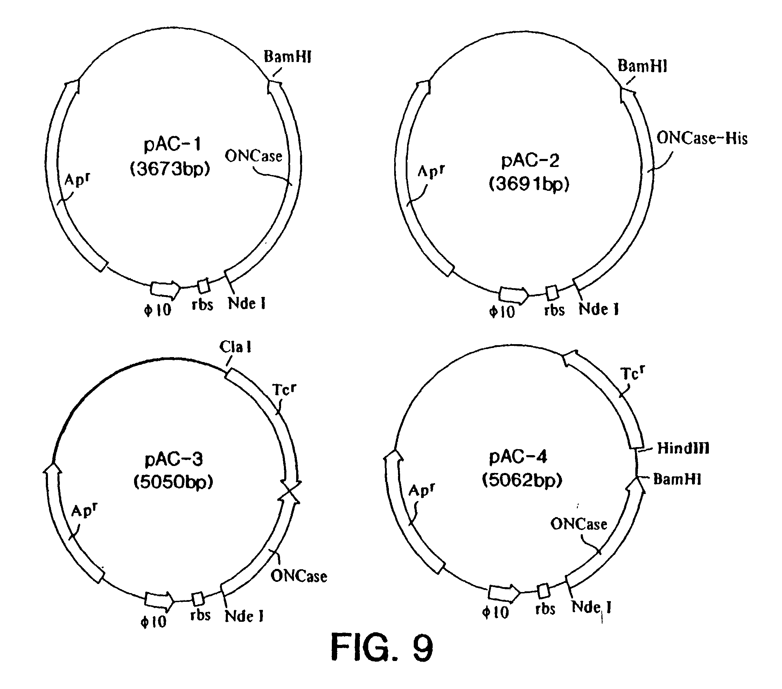

The Tetracycline-resistance gene was also obtained from pBR322 at the Ava I and Hind III sites. The Ava I end was filled into a blunt end, and was ligated with pAC-1 which was digested with the Hind III and Cla I restriction enzymes, with the Cla I end filled into a blunt end. Positive clones which became pink after storage at 4°C for 24 hours were selected from Tetracycline-containing plates. A high expression recombinant methioninase clone was determined by activity assay and named as the pAC-4 clone. A variety of high level expression clones are provided in Table 13 and Figure 9.

**Table 13**

| rMETase Expression Clones | | | | | |
|---|---|---|---|---|---|
| Clone | Vector | Antibiotic Resistance | Promoter | Fusion | Expression* (g/l) |
| pAC-1 | pT7.7 | Amp | T7 | --- | 1.0 |
| pAC-2 | pT7.7 | Amp | T7 | His. Tag | 0.5 |
| pAC-3 | pT7.7 | Tc | T7 | --- | 0.5 |
| pAC-4 | pT7.7 | Tc | T7 | --- | 1.0 |

| | | | | | |
|---|---|---|---|---|---|
| *Expression level in shaking flask (TB medium, 37°C, 400 rpm, 36 hours). | | | | | |

### Example 11

### Fermentation of Recombinant Methioninase Expression Clones

The expression clones of recombinant methioninase were grown in Terrific Broth medium containing either Ampicillin (100µg/ml) or Tetracycline (10µg), at 28°C or 37°C with 400 rpm shaking in a 6-L flask or fermenter.

### Example 12

### Purification of Recombinant Methioninase

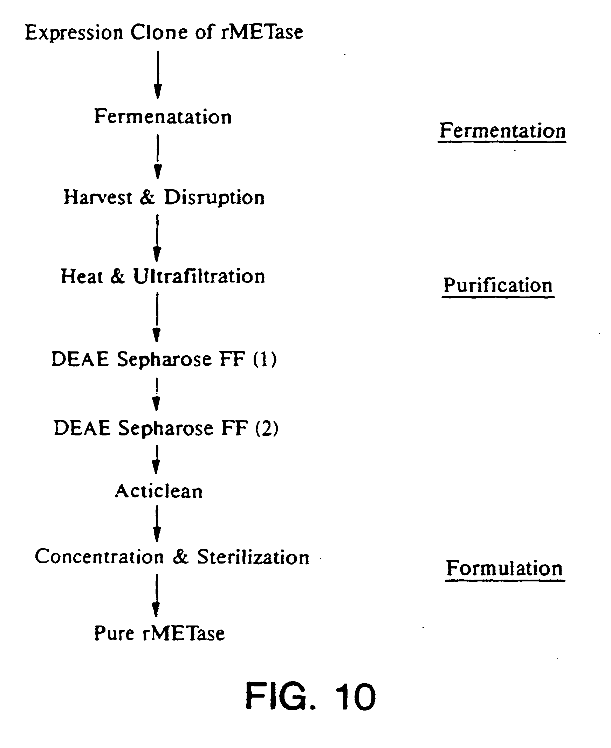

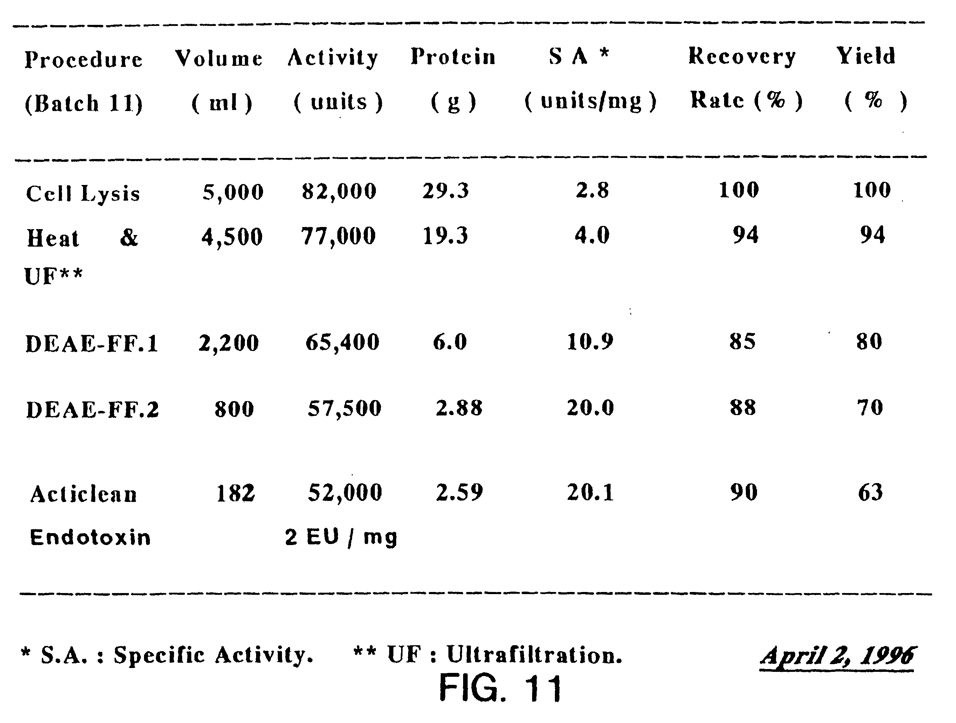

An outline of the purification method is provided in Figures 10 and 11.

### (1) Pre-column treatment of the sample

The bacteria were harvested by centrifugation at 800 x g at 4°C for 10 min. The bacterial pellet is then suspended in extraction solution (20mM potassium phosphate pH9.0, 10 µM pyridoxal phosphate and 0.01% β- mercaptoethanol) and disrupted with a cavitator -type homogenizer (Microfluidics Corp. model # HC 8000). Heat treatment of the homogenate is then carried out at 50°C for one minute. The suspension is centrifuged with an automatic refrigerated centrifuge (SORVALL Superspeed RC 2-B) at 4°C at 13 k rpm for 30 min. The supernatant is then collected. This step is followed by ultrafiltration by a Millipore Prep / Scale - TFF PLHK 100k 2.5 ft² cartridge with buffer (10mM potassium phosphate pH8.3). The pH is adjusted to 7.2 by ultrafiltration.

### (2) Chromatographic conditions

### The first column: DEAE Sepharose FF

- Column:: XK 100/60, Height: 32 cm, Volume: 2.5 L
- Solution:: [A] 40mM potassium chloride, 10mM potassium phosphate (pH7.2) containing 10 µM pyridoxal phosphate and 0.01% β - mercaptoethanol.
[B] 200mM potassium chloride, 10mM potassium phosphate (ph7.2) containing 10 µM pyridoxal phosphate and 0.01% β - mercaptoethanol.
- Flow Rate:: 5 ml/min.
- Sample:: About 100-200 g of total protein (10-20 mg/ml) are applied on the first column.
- Gradient:: [1] Pre-wash with solution A approximately 10 volumes until the OD₂₈₀ drops below 0.1.
[2] Gradient: Solution B from 20% - 100%.
- Fractions:: Elution fractions of 200 ml are collected. The fractions containing rMETase are identified by activity assay and pooled.

### The second column: DEAE Sepharose FF

- Column:: XK 50/30, Height: 25 cm, Volume: 500 ml
- Solution:: [A] 100mM potassium chloride, 10mM potassium phosphate (pH8.3) containing 10µM pyridoxal phosphate and 0.01% β - mercaptoethanol.
[B] 200mM potassium chloride, 10mM potassium phosphate (pH8.3) containing 10µM pyridoxal phosphate and 0.01% β - mercaptoethanol.
- Flow Rate:: 5 ml/min.
- Sample:: Approximately 10-20 g of total protein (2-4 mg/ml), after dialysis in 100mM potassium chloride, 10mM potassium phosphate (pH8.3) containing 10µM pyridoxal phosphate for 24 hours, are applied on the second column.
- Gradient:: [1] Pre-wash with solution A approximately 5 volumes until the OD₂₈₀ drops below 0.05.
[2] Gradient: Solution B from 0% - 60%.
- Fractions:: Elution fractions of 200 ml are collected. The fractions containing rMETase are identified by the activity assay and pooled.

### The third column: Sephacryl S-200 HR

- Column:: HiPrep 26/60, volume 320 ml.
- Solution:: 0.15 M sodium chloride in 10mM sodium phosphate (pH7.2)
- Flow Rate:: 1.2 ml/ min.
- Sample:: Approximately 10 ml concentrated sample. (after dialysis in 0.15 M sodium chloride, 10mM sodium phosphate (pH7.2) for 12 hours), are applied to the third column.
- Fractions:: Eluticn fractions of 20 ml containing rMETase, which are identified by yellow color and activity assay, are collected.

### The fourth column: Acticlean® Etox

Purified rMETase (10-20 mg protein / ml) in a volume of 100-200 ml is applied on a 500 ml Acticlean® Etox column, and eluted with elution buffer (0.15 M sodium chloride in 10mM sodium phosphate pH7.2) in order to eliminate endotoxin. Acticlean® Etox is reusable and can be cleaned with 1 M sodium hydroxide and can be autoclaved.

### Concentration of the final eluant

The final eluant is concentrate with 30 K Amicon Centriprep Concentrators. The formulation for purified rMETase is 0.15 M sodium chloride, 10mM sodium phosphate, pH7.2.

### Purification of rMETase.Histidine: Chromatography on Ni⁺⁺ Sepharose column

The cell homogenate, after pre-column treatment, is suspended in binding buffer (5mM imidazole, 0.5 M NaCl, 20mM Tris.HCL, pH7.9). The column is then washed with 10 volumes of binding buffer followed by washes with 6 volumes of wash buffer (60mM imidazole, 0.5 M sodium chloride, 20mM Tris, HCl, pH7.9). Elution occurs after 6 volumes of elution buffer (1 M imidazole, 0.5 M NaCl, 20mM Tris. HCl pH7.9) have been run through the column. The fractions containing rMETase, identified by yellow color, are collected.

### Example 13

### Analysis for The Purity of rMETase with HPLC

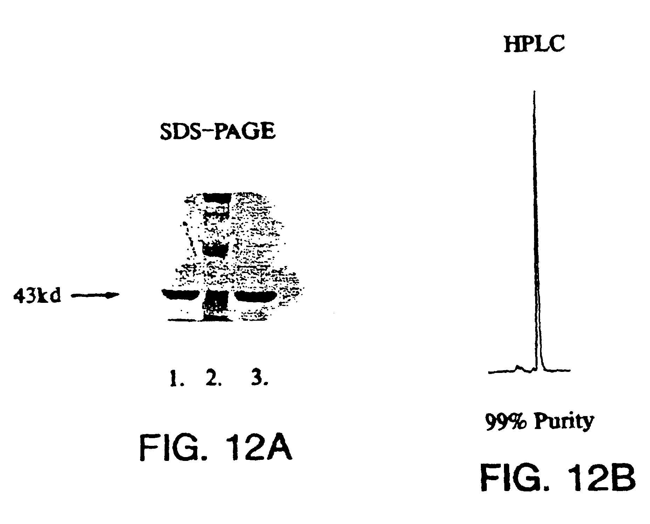

Column: SUPELCO, 8-08541, Progel TM - TSK, G 3000- SWXL, 30 cm x 7.8 mm.
Eluent Solution: 0.15 M sodium chloride in 10mM sodium phosphate buffer (pH7.2).
Flow Rate: 0.1 ml/min.
Sample: 20 µl (0.1 - 1 mg/ml).
An example for production of rMETase is shown in Figures 10 and 11. Purity is shown in Figure 12.

### Example 14

### Formulations Containing Recombinant Methioninase, Crystallized and Lyophilized Forms

### Solution formulation:

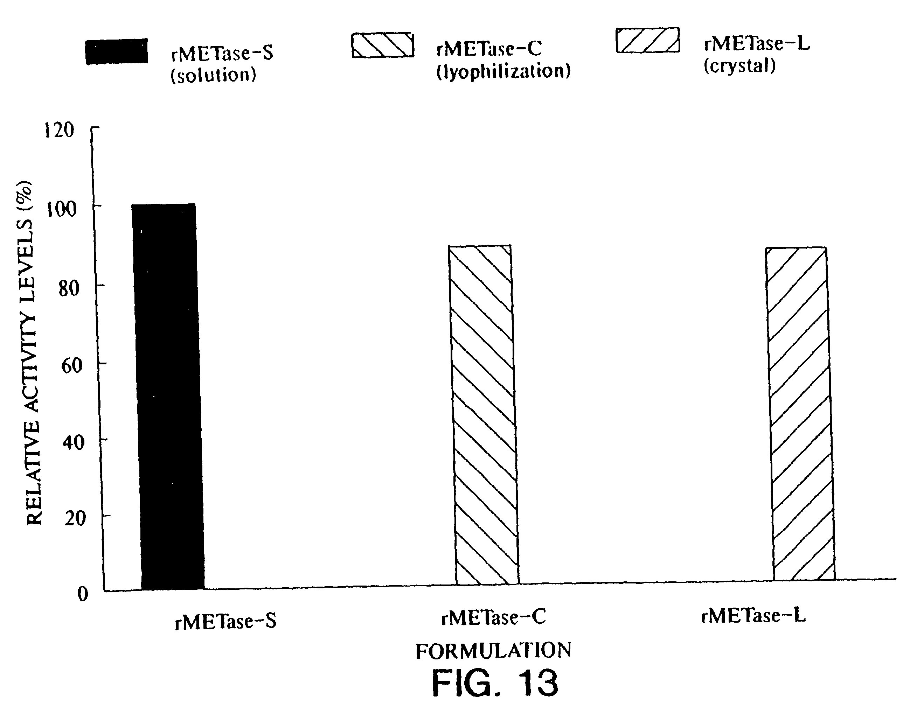

rMETase is formulated in solution, 0.15M sodium chloride, 10mM sodium phosphate buffer (pH 7.2), at the concentration 10-20mg/ml. The stability of rMETase is showed in Figure 13.

### Crystallized form:

rMETase (10-20mg/ml), in a 0.15 M sodium chloride and 10 mM sodium phosphate buffer (pH 7.2) was desalted using a Sephadex G-25 (DNA grade, superfine, Sigma) column. The solution was frozen on a dry ice and acetone bath and then crystallized in a vacuum of 100 milifar, at -80°C, for 72 hours using a Verdis Freeze Mobil 24.

### Lyophilized form:

rMETase (10-20mg/ml), in a 0.15 M sodium chloride and 10 mM sodium phosphate buffer (pH 7.2), was frozen on a dry ice and acetone bath and lyophilized in a vacuum of 100 milifar, at -80°C, for 72 hours using a Verdis Freeze Mobil 24.

### Assay for activity:

The assay was carried out in a 1 ml volume of 50 mM phosphate buffer pH 8.0, containing 10 µM pyridoxal phosphate and 10 mM methionine for 10 min. at 37°C with varying amounts of enzyme. The reaction was stopped by adding 0.5 ml of 4.5% TCA. The suspension was centrifuged at 15 K rpm for 2 min. 0.5 ml of supernatant with 0.5 ml of 0.05% 3-methyl-2-benzothiazolinone hydrazone in 1 ml of 1 M sodium acetate pH 5.2 was incubated at 50°C for 30 min. And α-Ketobutyrate was then determined by spectrophotometry at OD₃₃₅. The amount of protein was determined by the procedure of Lowry Reagent Kit (Sigma). The specific activity was calculated as units/mg protein.

The activity of rMETase were compared, and the results showed no big difference between different formulations.

### Example 15

### Chemical Modification of Recombinant Methioninase

The purified rMETase was formulated in a 0.15 M sodium chloride in 10 mM sodium phosphate buffer (pH 7.2) at a concentration between 0.1 M and 0.2 M. The activity was approximately 20 units/mg.

M-SC 5000 PEG molecular weight 5000 (Methoxy-SC-PEG, MW 5000 from Shearwater polymers Inc.), was dissolved in 20 mM sodium phosphate buffer (pH 8.3) at a concentration between 2mM and 20 mM. The molar rations of M-SC 5000 PEG to rMETase are varied from 10 : 1 to 120 : 1.

The PEGylation reactions were carried out in reaction buffer (25 mM sodium phosphate buffer, pH 8.3), at 20°C for 60 minutes. The reactions were stopped with stop buffer (0.14 M sodium phosphate buffer, pH 6.5) at 0°C. Unreacted M-SC 5000 PEG was then removed with 30 K Amicon Centriprep Concentrators. The resulting PEG - methioninase was formulated in 0.15 M sodium chloride and 10 mM sodium phosphate (pH 7.2) while centrifuging with 30k Amicon Centriprep concentrators.

### Analysis of PEG - rMETase in vitro

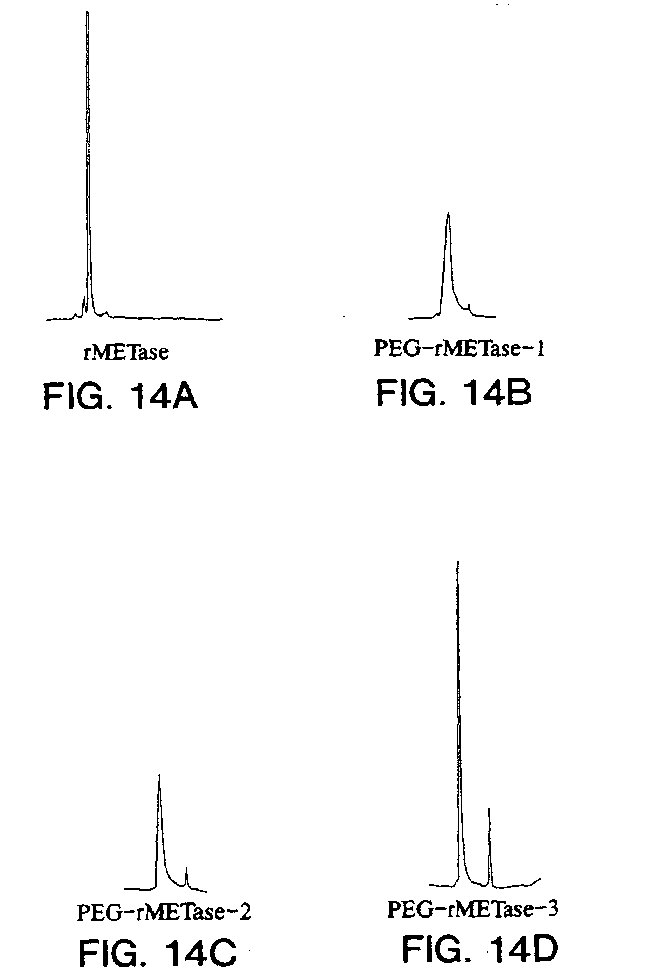

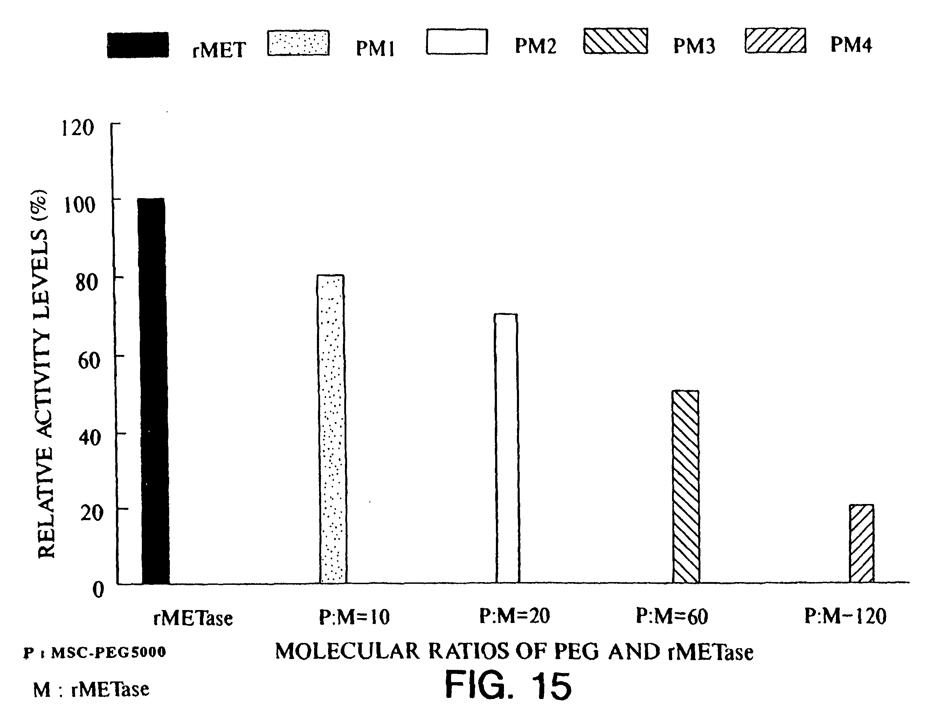

PEG-rMETase were analyzed by activity assay, electrophoresis and HPLC, Figures 14-16.

### Activity Assay

The activity of PEG-rMETase were between 80% to 20% of the unmodified rMETase.

### Electrophoresis

PEG-rMETase were applied to both native and SDS-PAGE.

### HPLC analysis:

PEG-rMETase were applied to a gel filtration column, no original rMETase peak was detected, only the PEG - METase peak were observed. The retention time (RT) were shorter along with the molecular ratios of PEG and rMETase increased.

### Pharmacokinetics of PEG-rMETase:

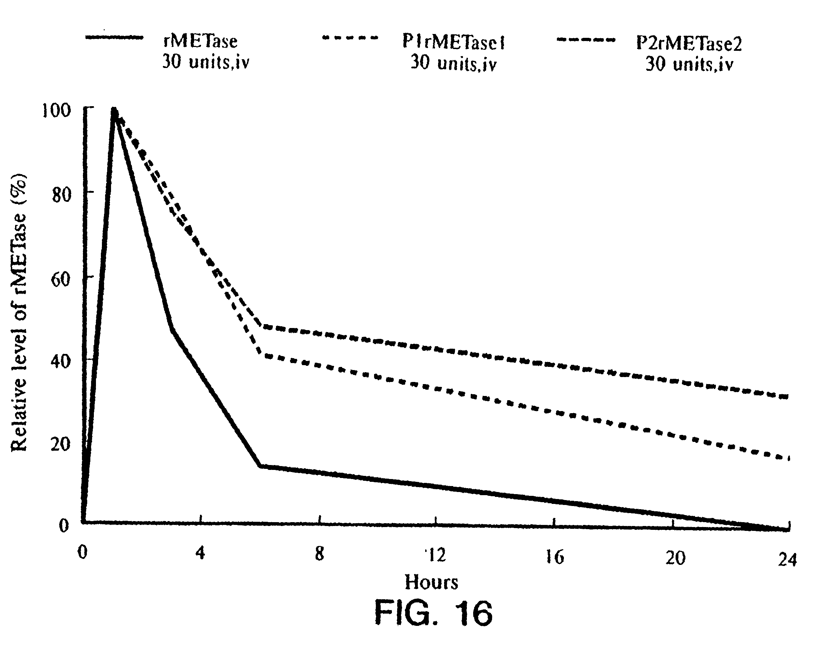

Purified endotoxin-free PEG-rMETase were injected into the tail-vein of mice. The blood samples were collected every two hours. The levels of rMETase were measured by activity assay (Figure 16).

### Example 16

### Efficacy and Toxicity of Recombinant Methioninase

### Growth Inhibition of Cells by rMETase in vitro

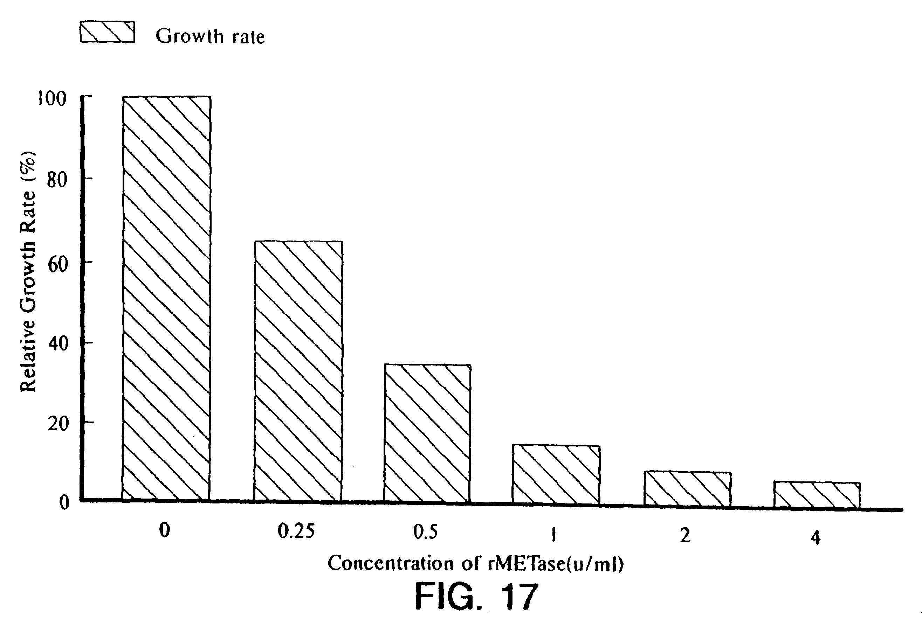

KB3-1 cells (Human squamous cell carcinoma) were grown in RPMI 1640 medium supplemented with 10% FBS. Various concentrations of rMETase were added to the medium and incubated at 37°C, 5% CO₂. The relative cell number was measured at OD₅₇₀. The results demonstrated that rMETase effectively inhibited cell growth (Figure 17).

### Growth Inhibition of H460 and HT29 by rMETase in vitro

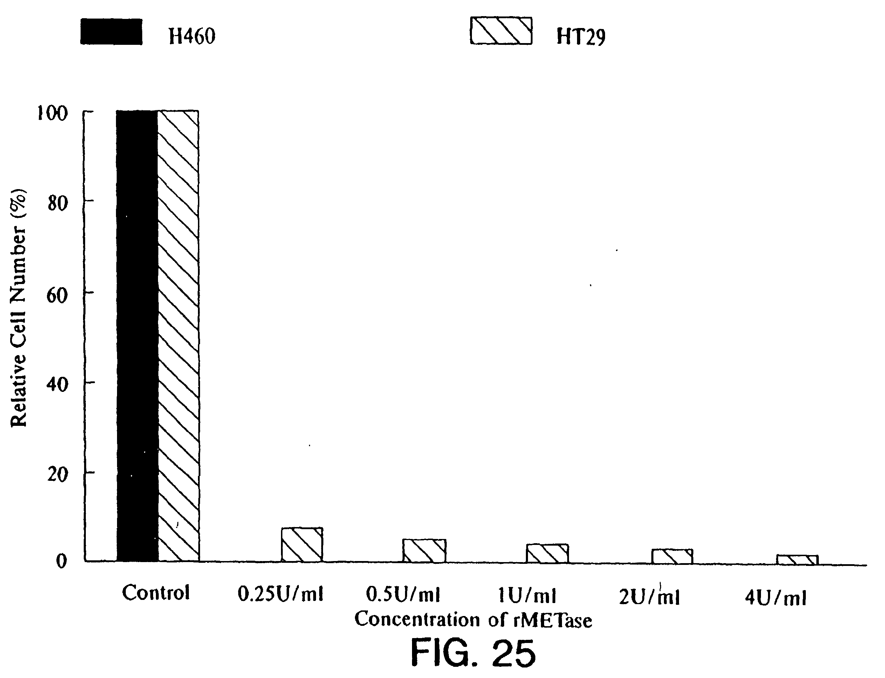

Human lung cancer cells H460 and Human colon cancer cells HT29 were grown with different concentrations of rMETase (0- 4 units/ml) for 4 days, then the live cells were counted. The experiments were repeated three times. The results demonstrated that rMETase effectively inhibited both H460 and HT29 cell growth (Fig 25).

### Comparisons of the sensitiveties to rMETase between normal cells and Human cancer cells in vitro

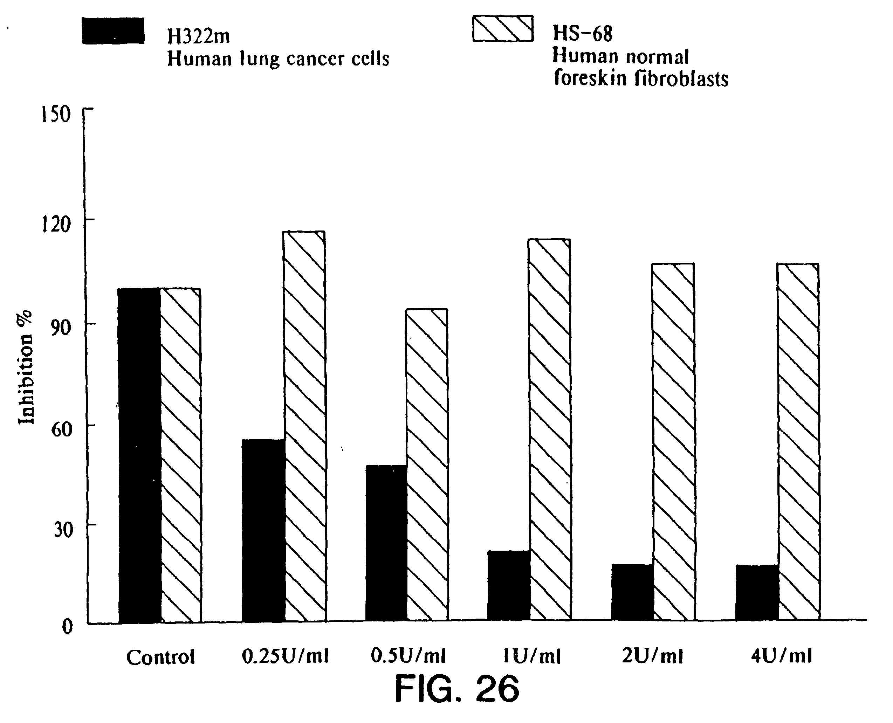

Normal human foreskin fibroblasts HS-68 and human keretinocytes were used as controls and compared with human colon cancer SW-620 and human lung cancer cells H322m. The cells were grown with different concentrations (0-4 units /ml) of rMETase for three days and then the live cells were counted. The experiments were repeated three times. The results showed that both SW- 620 and H322m cells were dead at the concentration 1 unit / ml of rMETase, the normal human HS-68 cells were still alive although the growth rate was reduced. The normal human keretinocytes were also much less sensitive to rMETase compared to cancer cells (Fig 26).

### Growth Inhibition of Cells by rMETase in Nude mice

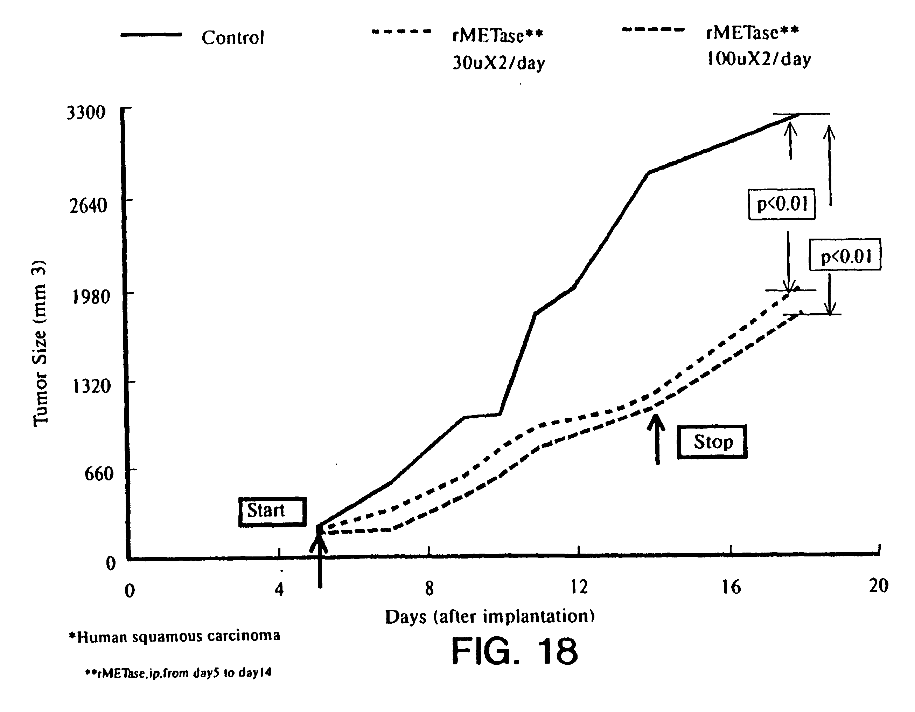

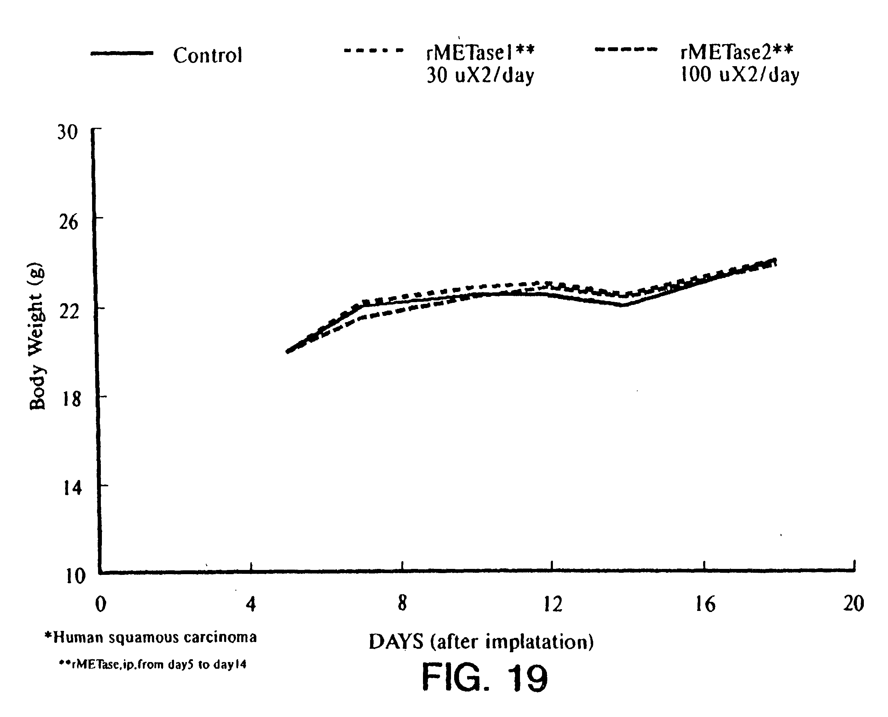

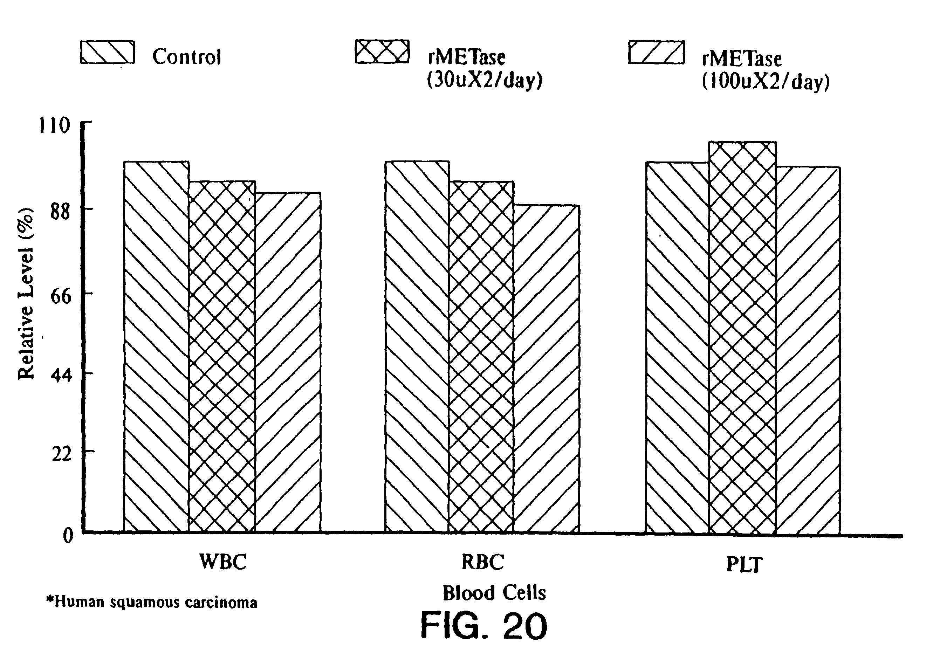

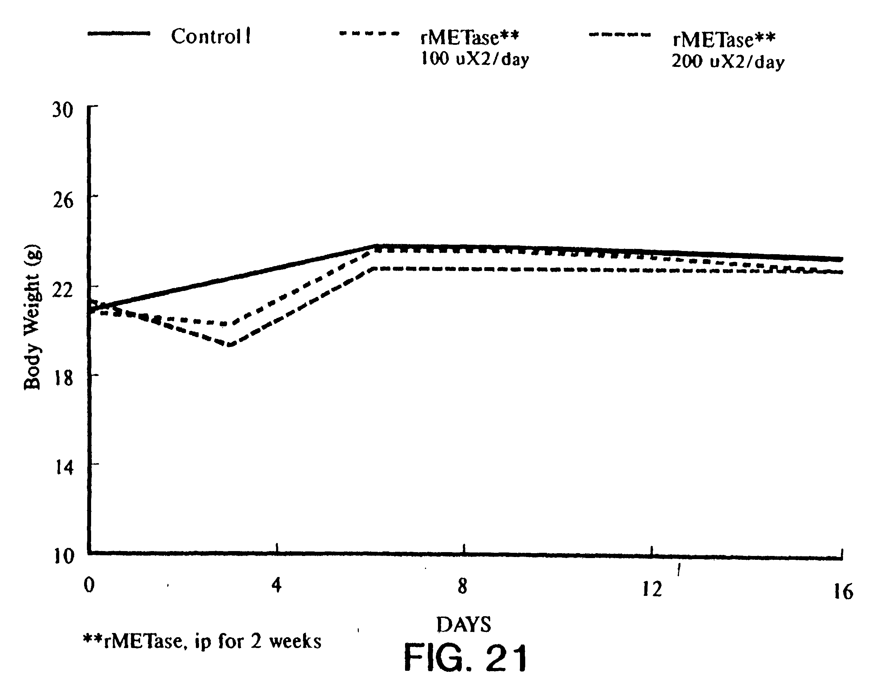

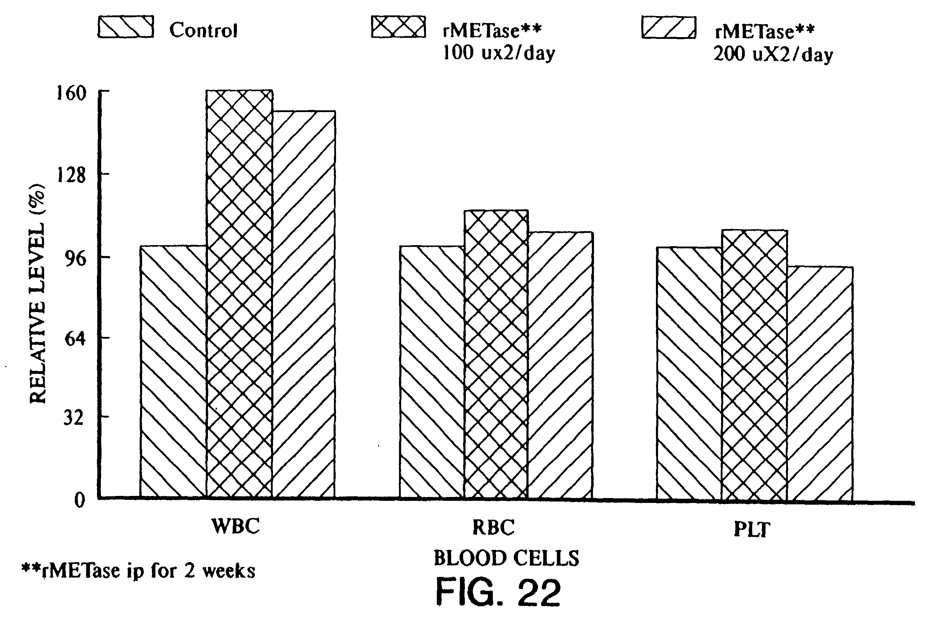

2 X 10⁵ cells were injected into Balb/c nu/nu, female, nice in groups of eight. Control: normal saline. Group I: rMETase 30 units, Group II: rMETase 100 units; ip twice a day from day 5 to day 14. The tumor size and body weight were measured. The blood was collected on day 18. The results demonstrated that rMETase effectively inhibited tumor growth without loss of body weight and without any effect on blood cell production (Figures 18-22).

### Growth Inhibition of H460 and HT29 Cells by rMETase in nude mice.

10⁶ H460 cells and HT29 cells were transplanted s.c., respectively, rMETase 40 units or 100 units twice a day were given by ip injection from day 2 to day 16. The control group had ip injection with normal saline 0.2 ml twice a day. The mice were sacrificed at day 16. The results demonstrated that rMETase effectively inhibited tumor growth without body weight loss and without effect on blood cell production.

### Pilot Phase I Clinical Trial of purified, recombinant METase

Patient 1, female, 50 years old, with stage IV breast carcinoma with lymph nodes metastasis, received 10,000 units (0.5g) rMETase iv infusion for 10 hours.

Patient-2, 48 years old, female, with state IV breast carcinoma with lymph nodes metastasis, received 5,000 units (0.25g) rMETase by in infusion for 24 hours.

Patient-3, 56 years old, female, with stage III renal carcinoma, received 10,000 units (0.5g) rMETase by iv infusion for 24 hours. Table 14.

Physical examinations were recorded and the blood samples were ccllected before treatment, during treatment every two hours and two hours and up to 48 hours (as indicated) after the end of the infusion. Laboratory determinations were carried out according to WHO criteria.

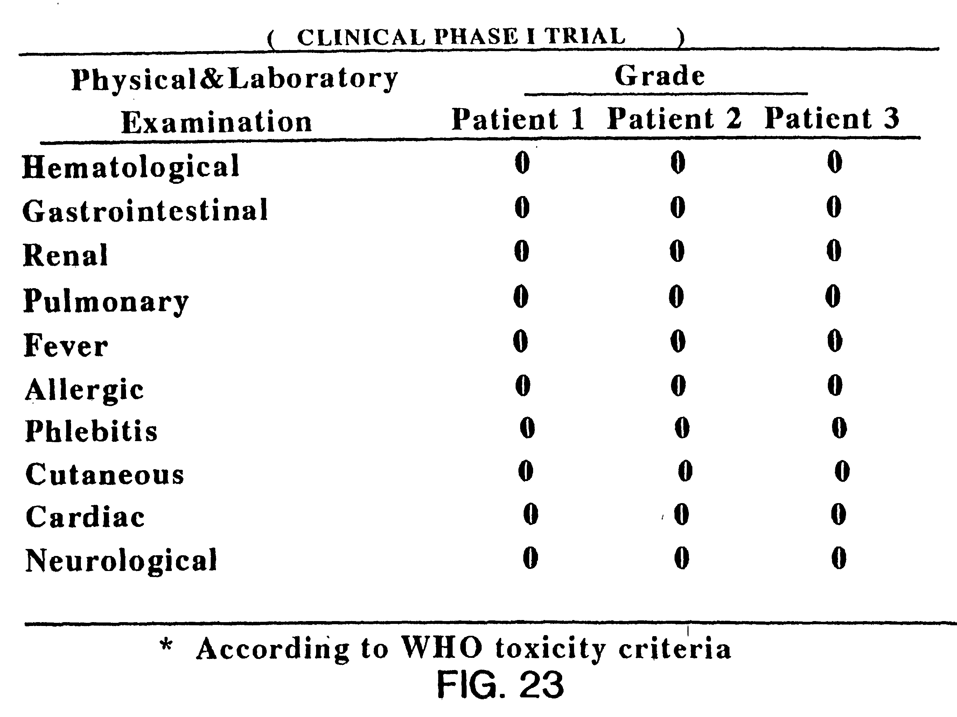

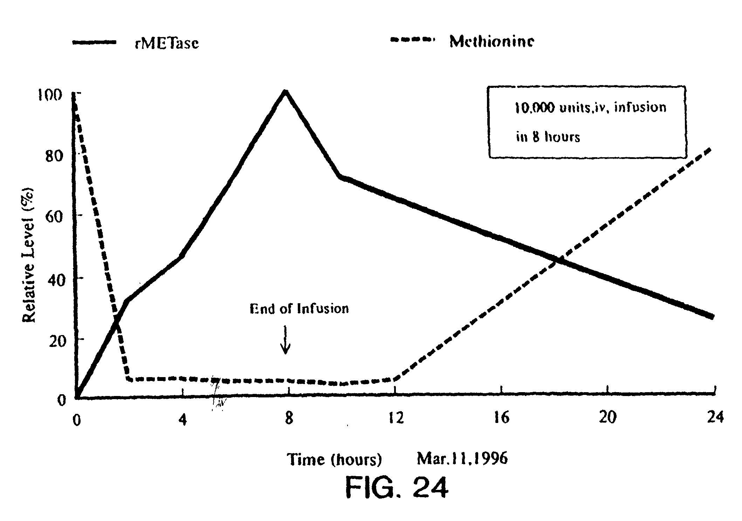

The results showed that the rMETase levels were enhanced immediately after the start of the infusion and maintained high level during the infusion in all patients. In one patient, after 48 hours, the methioninase level dropped back base line. The results showed that the rMETase level was enhanced immediately after the start of the infusion, reached the highest point at 10 hours. Eight hours after the infusion was stopped the level was 50% of the peak and was still maintained at 20% of the peak 16 hours after the end of the infusion. The results of the laboratory examinations were evaluated according to WHO criteria and showed no acute toxicity in patient-1, 2 or 3. Tables 15 and 16 and Figures 23 and 24. The result suggested that rMETase did not cause any toxicity

**Table 14**

| Protocol of Clinical Phase I Trial for Recombinant Methioninase | | | |
|---|---|---|---|
| | Patient I | Patient II | Patient III |
| Diagnosis | Breast cancer with metastasis | | Renal cancer |
| Sex | Female | Female | Female |
| Age | 46 | 48 | 56 |
| rMETase | 10.00 units | 5,000 units | 10.000 units |
| I.v. infusion | 8 hours | 24 hours | 24 hours |
| Blood collection | Before infusion and during infusion every two hours, after infusion 48 hours | | |
| Evaluation | WHO criteria | | |

**Table 15**

| Toxicity of Clinical Phase I Trial for Recombinant Methioninase | | | |
|---|---|---|---|
| Physical & Laboratory Examination | Grade | | |
| | Patient I | Patient II | Patient II |
| Hematological | 0 | 0 | 0 |
| Gastrointestinal | 0 | 0 | 0 |
| Renal | 0 | 0 | 0 |
| Pulmonary | 0 | 0 | 0 |
| Fever | 0 | 0 | 0 |
| Allergic | 0 | 0 | 0 |
| Phlebitis | 0 | 0 | 0 |
| Cutaneous | 0 | 0 | 0 |
| Cardiac | 0 | 0 | 0 |
| Neurological | 0 | 0 | 0 |

**Table 16**

| Pilot Phase I Clinical Trial, record of vital statistics in Patient I (rMETase 10.000 units iv infusion for 8 hours) | | | | | |
|---|---|---|---|---|---|
| Time | | | | | |
| | | Temp. | Pulse | respir. rate | Blood press. |
| Before infusion | | 36.70 | 61 | 20 | 114/76 |
| During infusion | 1h | 36.60 | 58 | 18 | 116/78 |
| | 2h | 36.60 | 59 | 20 | 118/60 |
| | 3h | 36.60 | 62 | 20 | 112/74 |
| | 4h | 36.70 | 64 | 20 | 112/74 |
| | 5h | 36.50 | 62 | 20 | 116/76 |
| | 6h | 36.60 | 64 | 20 | 116/78 |
| | 7h | 36.60 | 64 | 20 | 114/78 |
| | 8h | 36.60 | 62 | 20 | 114/76 |
| After infusion | 10h | 36.60 | 62 | 20 | 114/76 |
| | 24h | 36.60 | 60 | 18 | 114/76 |

## Claims

1. A modified methioninase comprising methioninase conjugated to polyethylene glycol,
wherein said modified methioninase maintains the high methioninase depletion activity of unmodified methioninase.

2. The modified methioninase of claim 1, wherein the methioninase is substantially free of endotoxin, and
has been recombinantly produced.

3. A therapeutic composition comprising a therapeutically effective amount of the modified methioninase of claim 1 or 2.

4. Use of a modified methioninase of claim 1 or 2 for the manufacture of a medicament for therapy.

5. Use of claim 4, wherein said medicament is for treating a tumor or cardiovascular disease.

6. Use of a modified methioninase of claim 1 or 2 for use in tumor diagnosis.

7. Recombinant host cells that contain a high-level expression module encoding methioninase, said module comprising a nucleotide sequence encoding methioninase operably linked to a promoter sequence, wherein said promoter sequence directs the expression of methioninase in said host cells such that the expression of methioninase is from 5-75% of the total protein produced by said host cells.

8. The cells of claim 7, wherein said methioninase-encoding nucleotide sequence is isolated from an organism selected from the group consisting of *Pseudomonas putida, Trichomonas vaginalis, Nippostrongylus brasiliensis*, and *Fusobacterium sp.*

9. The cells of claim 7 or 8 which are bacterial cells and wherein said promoter is the T7 RNA polymerase promoter.

10. The cells of claim 8 or 9, which are *E*. *coli* cells.

11. The cells of claim 10, which are *E*. *coli* BL21 (DE3) cells.

12. The cells of claim 7 or 8 which are immune cells.

13. The cells of claim 12 wherein the promoter is a tumor specific promoter.

14. A method to produce high levels of methioninase which method comprises culturing the cells of any of claims 8 to 13 and optionally recovering methioninase from said culture.

15. A method of identifying cells that express high levels of methioninase, said method comprising the steps of:
culturing said host cells under conditions wherein methioninase is expressed; and
identifying host cells that are pink in colour as producing high levels of methioninase.

16. The method of claim 15, wherein said cells are cultured on a solid medium.

17. A method of purifying methioninase, said method comprising the steps of:
(a) culturing the cells of any of claims 7 to 13 under conditions wherein methioninase is expressed;
(b) heating an extract of a transformed cell that contains methioninase in aqueous buffers from 40-60°C for 1-10 min.;
(c) centrifuging of the heated extract from 10k to 20k rpm [in a GS-3 rotor (Sorval Du Pont)] for 15 min. to 1 hour;
(d) subjecting the supernatant to ultrafiltration using a 50K to 100k pore size;
(e) subjecting the supernatant to DEAE ion exchange chromatography in low ionic strength (from 10-50mM) KCI at pH 7.0-7.6, and collecting fractions containing methioninase eluted in a 40-200mM KCI gradient;
(f) subjecting the fractions to a second DEAE ion exchange chromatography in medium ionic strength (100-200mM) KCI at pH 8.0-8.6, and collecting fractions containing methioninase eluted in a phosphate buffered 0.1 to 02M KCI;
(g) contacting said fractions collected in step (e) with a chromatography medium capable of absorbing endotoxin; and
(h) collecting the eluant containing methioninase.

## Patentansprüche

1. Modifizierte Methioninase umfassend an Polyethylenglykol konjugierte Methioninase,
worin genannte modifizierte Methioninase die hohe Methionin-Depletionsaktivität von nicht modifizierter Methioninase aufrechterhält.

2. Modifizierte Methioninase nach Anspruch 1, worin die Methioninase im Wesentlichen frei von Endotoxin ist und
rekombinant hergestellt wurde.

3. Therapeutische Zusammensetzung umfassend eine therapeutisch wirksame Menge der modifizierten Methioninase nach Anspruch 1 oder 2.

4. Verwendung einer modifizierten Methioninase nach Anspruch 1 oder 2 zur Herstellung eines Arzneimittels zur Therapie.

5. Verwendung nach Anspruch 4, worin genanntes Arzneimittel zur Behandlung eines Tumors oder einer kardiovaskulären Erkrankung bestimmt ist.

6. Verwendung einer modifizierten Methioninase nach Anspruch 1 oder 2 zur Verwendung bei der Tumordiagnose.

7. Rekombinante Wirtszellen, die einen hohen Anteil des Expressionsmoduls codierend Methioninase enthalten, wobei genannter Modul eine Nukleotidsequenz codierend Methioninase, die funktionsfähig mit einer Promotorsequenz verknüpft ist, umfasst, worin genannte Promotorsequenz die Expression von Methioninase in genannte Wirtszellen dergestalt richtet, dass die Expression von Methioninase von 5 - 75 % des von genannten Wirtszellen produzierten Gesamtproteins beträgt.

8. Zellen nach Anspruch 7, worin genannte Methioninase-codierende Nukleotidsequenz aus einem Organismus isoliert wird, der aus der Gruppe ausgewählt ist, bestehend aus *Pseudomonas putida, Trichomonas vaginalis, Nippostrongylus brasiliensis* und *Fusobacterium sp*.

9. Zellen nach Anspruch 7 oder 8, die Bakterienzellen darstellen und worin genannter Promotor der T7-RNA-Polymerase-Promotor ist.

10. Zellen nach Anspruch 8 oder 9, die Zellen von *E*. *coli* darstellen.

11. Zellen nach Anspruch 10, die Zellen von *E*. *coli* BL21 (DE3) darstellen.

12. Zellen nach Anspruch 7 oder 8, die Immunzellen darstellen.

13. Zellen nach Anspruch 12, worin der Promotor einen Tumor-spezifischen Promotor darstellt.

14. Verfahren zur Bildung hoher Methioninase-Spiegel, welches Verfahren das Kultivieren der Zellen nach einem der Ansprüche 8 bis 13 und optional die Wiedergewinnung von Methioninase aus genannter Kultur umfasst.

15. Verfahren zur Identifikation von Zellen, die hohe Methioninase-Spiegel exprimieren, wobei genanntes Verfahren die folgenden Schritte umfasst:
Kultivieren genannter Wirtszellen unter Bedingungen, worin Methioninase exprimiert wird und
Identifizieren von Wirtszellen, die rosafarbig sind, als Bildner hoher Methioninase-Spiegel.

16. Verfahren nach Anspruch 15, worin genannte Zellen auf einem Festmedium kultiviert werden.

17. Verfahren zur Reinigung von Methioninase, wobei genanntes Verfahren die folgenden Schritte umfasst:
(a) Kultivieren der Zellen nach einem der Ansprüche 7 bis 13 unter Bedingungen, worin Methioninase exprimiert wird;
(b) Erhitzen eines Extrakts aus einer transformierten Zelle, die Methioninase in wässrigen Puffern enthält, von 40 - 60 °C für 1 - 10 min;
(c) Zentrifugieren des erhitzten Extrakts von 10k bis 20k U/min [in einem GS-3-Rotor (Sorval DuPont)] für 15 min bis 1 Stunde;
(d) Aussetzen des Überstandes der Ultrafiltration unter Verwendung einer Porengröße von 50k bis 100k;
(e) Aussetzen des Überstandes der DEAE-Ionenaustausch-Chromatographie in KCI einer niedrigen Ionenstärke (von 10 - 50 mM) bei pH 7,0 - 7,6 und Sammeln der Fraktionen enthaltend Methioninase, die mit einem KCl-Gradienten von 40 - 200 mM eluiert werden;
(f) Aussetzen der Fraktionen einer zweiten DEAE-Ionenaustausch-Chromatogrpahie in KCI einer mittleren Ionenstärke (100 - 200 mM) bei pH 8,0 - 8,6 und Sammeln der Fraktionen enthaltend Methioninase, die in Phosphat-gepufferter KCl von 0,1 bis 0,2 M KCI eluiert werden;
(g) Kontaktieren genannter Fraktionen, die in Schritt (e) mit einem zum Absorbieren von Endotoxin fähigen Chromatographie-Medium gesammelt werden; und
(h) Sammeln des Methioninase enthaltenden Eluenten.

## Revendications

1. Méthioninase modifiée comprenant de la méthioninase conjuguée à du polyéthylène glycol,
dans laquelle la méthioninase modifiée maintient l'activité d'appauvrissement en méthionine élevée de la méthioninase non modifiée.

2. Méthioninase modifiée selon la revendication 1, dans laquelle la méthioninase ne contient pratiquement pas d'endotoxine, et
a été produite de façon recombinante.

3. Composition thérapeutique comprenant une quantité thérapeutiquement efficace de méthioninase modifiée selon la revendication 1 ou 2.

4. Utilisation d'une méthioninase modifiée selon la revendication 1 ou 2 pour la fabrication d'un médicament destiné à une thérapie.

5. Utilisation selon la revendication 4, dans laquelle ledit médicament est utilisé pour le traitement d'une tumeur ou d'une maladie cardiovasculaire.

6. Utilisation d'une méthioninase modifiée selon la revendication 1 ou 2 destinée à être utilisée dans le diagnostic d'une tumeur.

7. Cellules hôtes recombinantes qui contiennent un module à haut niveau d'expression codant la méthioninase, ledit module comprenant une séquence de nucléotides codant la méthioninase liée de façon opératoire à une séquence du promoteur, dans lequel ladite séquence du promoteur contrôle l'expression de la méthioninase dans lesdites cellules hôtes de sorte que l'expression de la méthioninase représente de 5 à 75 % de la protéine totale produite par lesdites cellules hôtes.

8. Cellules selon la revendication 7, dans lesquelles ladite séquence de nucléotides codant la méthioninase est isolée à partir d'un organisme choisi dans le groupe constitué par *Pseudomonas putida, Trichomonas vaginalis, Nippostrongylus brasiliensis* et *Fusobacterium sp*.

9. Cellules selon la revendication 7 ou 8, lesquelles sont des cellules bactériennes ou dans lesquelles ledit promoteur est le promoteur de l'ARN polymérase T7.

10. Cellules selon la revendication 8 ou 9, lesquelles sont des cellules de *E*. *coli*.

11. Cellules selon la revendication 10, lesquelles sont des cellules d'*E*. *coli* BL21 (DE3).

12. Cellules selon la revendication 7 ou 8, lesquelles sont des cellules immunologiquement compétentes.

13. Cellules selon la revendication 12, dans lesquelles le promoteur est un promoteur spécifique de tumeur.

14. Procédé de production de concentrations élevées de méthioninase, lequel procédé comprenant la culture des cellules selon l'une quelconque des revendications 8 à 13 et éventuellement la récupération de la méthioninase à partir desdites cultures.

15. Procédé d'identification des cellules qui expriment des concentrations élevées de méthioninase, ledit procédé comprenant les étapes consistant à :
cultiver lesdites cellules hôtes dans des conditions dans lesquelles la méthioninase est exprimée ; et
identifier les cellules hôtes qui sont de couleur rose comme elles produisent des concentrations élevées de méthioninase.

16. Procédé selon la revendication 15, dans lequel lesdites cellules sont cultivées sur un milieu solide.

17. Procédé de purification de la méthioninase, ledit procédé comprenant les étapes consistant à :
(a) cultiver les cellules de l'une quelconque des revendications 7 à 13 dans des conditions dans lesquelles la méthioninase est exprimée ;
(b) chauffer un extrait d'une cellule modifiée qui contient la méthioninase dans des solutions tampons aqueuses de 40 à 60°C pendant 1 à 10 minutes ;
(c) centrifuger l'extrait chauffé de 10000 à 20000 rpm [dans un rotor GS-3 (Sorval Du Pont)] pendant 15 minutes à 1 heure ;
(d) soumettre le surnageant à l'ultrafiltration en utilisant une taille de pores de 50K à 100k;
(e) soumettre le surnageant à une chromatographie par échange d'ions sur colonne DEAE dans du KCl (de 10 à 50 mM) de faible force ionique à un pH de 7,0 à 7,6 et recueillir les fractions contenant la méthioninase éluée dans un gradient de KCl de 40 à 200 mM ;
(f) soumettre les fractions à une seconde chromatographie par échange d'ions sur colonne DEAE dans du KCl (de 100 à 200 mM) de force ionique moyenne à un pH de 8,0 à 8,6 et recueillir les fractions contenant la méthioninase éluée dans une solution de KCl de 0,1 à 0,2 M tamponnée avec du phosphate ;
(g) mettre en contact lesdites fractions recueillies dans l'étape (e) avec un milieu pour chromatographie capable d'absorber l'endotoxine ; et
(h) recueillir l'éluant contenant la méthioninase.
